# EUROPEAN PATENT SPECIFICATION

(11) **EP 1 485 140 B1**
(45) Date of publication and mention of the grant of the patent: **26.07.2006**
(21) Application number: 03712334.6
(22) Date of filing: 11.03.2003
(51) Int. Cl.: A61L 27/38, A61L 27/56, A61K 9/16

(54) **POLYMER COMPOSITE LOADED WITH CELLS**
POLYMERKOMPOSITE MIT ZELLEN
COMPOSITION DE POLYMERE CHARGEE DE CELLULES

(30) Priority: 13.03.2002 GB 0205867
(43) Date of publication of application: 15.12.2004
(73) Proprietor: The University of Nottingham, Nottingham NG7 2RD (GB)
(72) Inventor: HOWDLE, Steven Melvyn, University of Nottingham, Nottingham NG7 2RD (GB); SHAKESHEFF, Kevin Morris, University of Nottingham, Nottingham NG7 2RD (GB); WHITAKER, Martin James, University of Nottingham, Nottingham NG7 2RD (GB); ROSE, Felicity Rosamari, University of Nottingham, Nottingham NG7 2RD (GB)
(74) Representative: Snodin, Michael D.
(86) International application number: PCT/GB2003/000999
(87) International publication number: WO 2003/077965

(56) References cited:
- WO-A-02/00275
- WO-A-91/09079
- HILE D D ET AL: "Active growth factor delivery from poly(d,l-lactide-co-glycolide) foams prepared in supercritical CO2" JOURNAL OF CONTROLLED RELEASE, ELSEVIER SCIENCE PUBLISHERS B.V., AMSTERDAM, NL, vol. 66, no. 2-3, May 2000 (2000-05), pages 177-185, XP004193066 ISSN: 0168-3659
- ANGELA K, DILLOW, FARIBA DEHGHANI, JEFFREY S. HRKACH, NEIL R. FOSTER AND ROBERT LANGER : "Bacterial inactivation by using near- and supercritical carbon dioxide" PROC. NATL. ACAD. SCI. USA, vol. 96, 1999, pages 10344-10348, XP002246774 cited in the application

## Description

The present invention relates to a process for the preparation of a polymer composite comprising contacting polymer with plasticising fluid and functioning matter and isolating loaded polymer, the polymer composite obtained thereby, and apparatus for the preparation thereof, a polymer scaffold, delivery device or the like comprising the composite in suitably sized and shaped form, the use as a pharmaceutical or veterinary product, a human or animal health or growth promoting, structural, fragrance or cosmetic product, an agrochemical or crop protection product, in biomedical, catalytic and like applications, more particularly as a surgical implant, synthetic bone composite, organ module, and the like or for bioremediation, as a biocatalyst or biobarrier and the like.

The use of supercritical fluids in the production of polymers as a plasticising, foaming or purification agent is known. Supercritical fluids (SCFs) act as plasticisers for many polymers, increasing the mobility of the polymer chains. This results in an increase in the free volume within the polymeric material.

Supercritical fluids have found application in incorporation of dyes and other inorganic materials which are insoluble in the supercritical fluid, for example inorganic carbonates and oxides, into polymers with a good dispersion to improve quality, in particular dispersion in products such as paints for spray coating and the like.

Moreover the fluid can be used to foam the polymer by transition to non-critical gaseous state whereby a porous material may be obtained and this has been disclosed in US 5,340,614, WO91/09079 & US 4,598,006.

SCFs also act as a solvent for impurities (including unreacted monomer and residual conventional solvents) which may be removed during the processing to give high purity products.

SCFs are also of widespread use in cell-breaking for extraction of materials from natural sources such as subcellular matter from mammalian and plant cells and other organic matter, usually involving pulverising the natural source matter i.e. breaking cells in the process of extraction. This is an established and growing technology which is particularly effective for mammalian cells.

Polymers have also been used in biomedical applications to develop materials in which biocompatibility can be influenced to promote favourable tissue responses whilst also producing materials with acceptable mechanical and surface properties. Biofunctional composite materials e.g. calcium hydroxyapatite dispersed in various polymers are well established for orthopaedic, dental and other applications. These materials are prepared with very high loadings of biofunctional inorganic solid, of up to 80%, in the form of a powder, and a composite is formed either by vigorous mixing of the powdered material into the solid or molten polymer, or by polymerisation of the monomers in the presence of suspended inorganic powders. In both cases, the material becomes entrapped within the polymer matrix.

WO 98/51347 (Howdle et al) discloses the preparation by SCF processing of biofunctional polymers comprising biofunctional material having the desired mechanical properties both for commercial processing and for implant into a human or animal host structure such as bone or cartilage, dental and tissue structures into which they are surgically implanted for orthopaedic implant, prosthetic, dental filling or restorative applications, prolonged release applications and the like. Biofunctional material is in particular any pharmaceutical, veterinary, agrochemical, human and animal health and growth promoting, structural, cosmetic and toxin absorbing materials, such as a broad range of inorganic and organic molecules, peptides, proteins, enzymes, oligosaccharides, carbohydrates, nucleic acids and the like.

In the Examples, various materials are introduced, including in a particular example the protein catalase which is processed at 45C under supercritical conditions. Subsequent assay showed retention of enzyme activity. This work is disclosed in more detail in Chem Commun. 2001, 109-110, Howdle et al.

This and other work from the same authors has established that biologically active materials can be mixed with polymers plasticised in SCF in the absence of additional solvent, yet retaining activity of the biological materials.

US 5766637 (Shine et al) also discloses introducing biologically active materials into polymers plasticised in supercritical fluid, and gives as sole example the incorporation of a vaccine comprising a virus. Viruses are robust to many conditions including (re)freezing. The virus is first cooled by refrigeration (implying temperature in excess of 0C or in excess of 3C or 4C), then contacted with SCF for a prolonged period wherein temperature is held at 22°C for 2 hours then increased to 37C, during which time the polymer is processed. Pressure is then released and the virus loaded polymer is then frozen.

Polymers are also being developed in biomedical applications as biological cell-laden scaffolds for use as biomedical inserts such as bone inserts, and as organ and tissue modules for *in vitro* and *in vivo* use, as inserts or for *in vivo* studies. A considerable effort is being invested in developing scaffolds which encourage growth and development of particular types of living matter and in particular configurations to mimic living systems. Cell-loading is typically by dropping a cellular soup onto the scaffold surface and allowing to permeate in whereby cells are seeded and are found to grow and proliferate into and throughout the scaffold itself on culturing. This is relatively time consuming, the rate limiting factor being the cell growth and proliferation, moreover each scaffold must be configured for the desired growth configuration.

It is an object of the present invention to provide a method for loading cells into polymers directly, i.e. for instant production of cell-laden scaffolds, in one step, in contrast with forming a scaffold and seeding with cells. To date no one has found a means to achieve this, and keeping the cells alive.

"Bacterial inactivation by using near- and supercritical carbon dioxide" Dillow *et al,* Proc. Natl. Acad. Sci. USA, Vol 96 pp 10344-10348, Aug 1999 discloses contacting bacterial cells with SC CO₂ under supercritical conditions of 25 - 35C, 2.05 x 10⁷ Nm⁻² (205 bar) in the absence and presence of polymer microspheres. Contact times of 0.1 to 4 hours, typically 30 and 45 minutes were employed . Contacting was reported to achieve bacterial inactivation and thus sterilisation of polymer where present. Sterilisation and virus inactivation kits using SCF are available.
We have now surprisingly found, contrary to the indications of Dillow, that the properties of plasticising fluids in general may be employed in the preparation of polymer composites comprising loaded cells and the like distributed throughout the composite, in manner that loaded populations of cells and the like remain viable after processing. The inventors have found that cryopreserved cell pellets can be exposed to carbon dioxide under plasticising conditions without interacting with the fluid. The inventors have also otherwise preserved non-cryopreserved cells against the harmful effects of plasticising carbon dioxide by imposing temperature and pressure constraints. In each case cells have been found to remain viable on thawing or on regaining ambient conditions after exposure to plasticising carbon dioxide. Significantly the inventors have found that cell pellets can be exposed to plasticising fluid under plasticising conditions and still maintain viability provided that, if frozen prior to contacting, cells remain frozen or thaw but are not allowed to thaw and refreeze, or if not frozen prior to contacting, cells are not allowed to freeze during contacting. Certain effects are also thought to be associated with certain fluids, for example temperature and pressure constraints must be applied when contacting cells with carbon dioxide in a particular temperature range at which dry ice formation may take place. Accordingly we have found that conditions may be varied by selection of plasticising fluid, or mixture thereof, in manner to provide a polymer composite loaded with cells with retention of viability.

This is particularly surprising since cells are highly sensitive to a number of conditions, including elevated temperature, contact with solvents, and lack of nutrients and oxygen. Moreover the established prior art use of super critical fluid in cell-breaking and for sterilisation of materials by inactivating bacterial cells teaches away from the present finding.

Accordingly in the broadest aspect of the invention there is provided a process for the preparation of a polymer composite loaded with functioning matter wherein the process comprises contacting a polymer substrate and an amount of functioning matter with a plasticising fluid, or mixture of plasticising fluids, under plasticising conditions for a period sufficient to plasticise and/or swell the polymer and incorporate the functioning matter, and releasing the plasticising fluid to obtain the polymer composite, wherein contacting is at a pressure in the range 1 x 10⁵ Nm⁻² to 1 x 10⁸ Nm⁻² (1 to 1000 bar) and a temperature in the range -200 to +500°C selected in manner that at least a proportion of functioning matter does not freeze or refreeze during processing, or if at a temperature at which freezing or refreezing may occur, that either matter is desiccated or a pressure constraint is applied, whereby pressure is in a range having a maximum of less than 1 x 1.0⁸ Nm⁻² (1000 bar) throughout contact of functioning matter and plasticising fluid, whereby at least a proportion of functioning matter retains its function in the polymer composite. For example we have observed that freezing or refreezing may take place during processing with carbon dioxide as plasticising fluid at least in the temperature range +4 to +35°C.

Reference herein to functioning matter is to matter which under favourable conditions is capable of performing a function, such as growth, movement, metabolism of substances, generation of substances for example, or other functioning typically attributed with living matter and whose function is reversibly or irreversibly prejudiced under non favorable conditions. Functioning matter according to the invention may not be actively functioning at all times but may be dormant or inactive at any given time, and in particular is unlikely to be actively functioning during the process of the invention. Functioning matter may be chemically or physically preserved prior to contact with plasticising fluid against harmful effects associated with the plasticising fluid, preferably is preserved in dry form, for example may be cryopreserved or desiccated as hereinbelow defined. Functioning matter may be maintained in preserved state throughout contacting or may be allowed to revert to non preserved state depending on the prevailing conditions. Alternatively matter is physically preserved by imposing a pressure constraint which has been found to preserve against harmful effects associated with the plasticising fluid.
Alternatively functioning matter may be hydrated, ie non frozen and non desiccated.

Preferably contacting is with cryopreserved or non cryopreserved functioning matter and is conducted at a temperature in a range at which functioning matter remains frozen or unfrozen, or thaws without refreezing during contacting or is with cryopreserved or non-cryopreserved functioning matter and is conducted at a temperature at which freezing or refreezing may occur and with pressure constraint less than a maximum pressure as hereinbefore defined.

Preferably when contacting is at a temperature in the range substantially +4°C to substantially 35°C functioning matter is not preserved in dry form but by imposing pressure constraint.

Preferably the process for the preparation of a polymer composite loaded with functioning matter comprises contacting a polymer substrate and an amount of functioning matter with a plasticising fluid or mixture of plasticising fluids, in particular carbon dioxide or a mixture thereof, under plasticising conditions for a period sufficient to plasticise and/or swell the polymer and incorporate the functioning matter, and releasing the plasticising fluid to obtain the polymer composite, wherein contacting is with cryopreserved or non cryopreserved functioning matter and is conducted at a temperature in a range at which functioning matter remains frozen or unfrozen, or thaws without refreezing during contacting, for example -100 to +4 °C, +35 to +100°C or is with cryopreserved or non cryopreserved functioning matter and is conducted at a temperature at least in the range +4 to +35°C and maximum pressure less than 1 x 10⁸ Nm⁻² (1000 bar), which may be less than 4 x 10⁷ Nm⁻² (400 bar) or 2.75 x10⁷ Nm⁻² (275 bar), for example in the range 5x10⁵ Nm⁻² to 75x10⁵ Nm⁻² (5 to 75 bar) throughout contact of functioning matter and plasticising fluid, whereby functioning matter retains its function in the polymer composite.

Reference herein to functioning matter is to matter capable of performing or exhibiting specific functioning, and whose functioning is harmed by contact with plasticising fluid under plasticising conventional conditions. For example SCF dissolves or diffuses into biological cells and like enclosed fluid matter such as liposomes with harmful effect on cell functioning or liposome content functioning. Biological cells comprise liquid contents and SC CO₂ can easily penetrate, permeate and cause damage to these contents.

Chemical or physical preservation according to the invention preferably comprises rendering functioning matter dormant, and therefore is reversible. Preferably preservation comprises rendering functioning matter in plasticising fluid-impenetrable state and/or isolating functioning matter contents which facilitate plasticising fluid damage.
Preferably functioning matter is non cryopreserved, ie non frozen or is physically preserved by cryopreservation, i.e. freezing, preferably at a temperature in the range -10 to -100°C, most preferably - 20 to - 75°C.

Techniques for cryopreservation are known in the art, for example the matter may be immersed in liquid nitrogen or dry ice containing solvent such as ethanol, or enveloped in dry ice or convection cooled. Cryopreservation agents are suitably employed.

Alternatively functioning matter is physically preserved by desiccating for example by contact with a desiccant, preferably trehalose. Desiccation is determined according to known techniques, by weight loss, to agree with the calculated water content. Preferably desiccation is substantially 100%, for example 70% to 100%. Desiccation is at reduced pressure as known in the art. Water which facilitates damage in the presence of plasticising fluid is thereby removed.

By means of preservation in dry form by desiccation or freezing the liquid contents of the biological cell, for example, are rendered solid and therefore less likely to be harmed by CO₂, and moreover the cell is less likely to be penetrated by CO₂.

Matter may conveniently be preserved immediately prior to processing, eg 2 hours prior to processing, or may be preserved and stored for periods of the order of 1 week or more prior to processing. Matter may be provided already in preserved state.

Reference herein to loading polymer composite is to introducing matter internally in uniform or random distribution, throughout the composite cross-section, otherwise known as 3D distribution or internal distribution. Loading may be in a preliminary amount in the case of matter which is capable of subsequent growth to a desired final loading.

Loading may be of any desired polymer volume selected according to desired product quantities and also processing time, as hereinbelow defined. Suitable polymer volumes may be small e.g. 5 or 10g polymer, up to multi kg scale.

Preferably functioning matter is provided in dry or wet particulate or powder form suitable for loading into the polymer, and may be of particle size in the range 1 micron to 1 cm, preferably 50 micron to 1000 micron in case of biological cells and aggregates. Functioning matter may be of uniform or non-uniform particle size, depending on practical constraints and the required loading uniformity and distribution. Particles may be used dry or in suspension in a carrier or other agent, preferably a cryopreservation agent.

The process of the invention may be conducted in manner that functioning matter preserves its function entirely or in part depending on the requirements of the product composite and on the nature of the functioning matter. Suitably at least a proportion of functioning mater maintains substantially full viability for example at least 20%, preferably at least 40%, more preferably at least 60% up to substantially 100% of functioning matter maintains full function. In the process of the invention it is simple to load a composite with an amount of functioning matter allowing for wastage whereby a proportion thereof may lose function leaving a proportion as hereinbefore defined with maintained function. Optionally the composite comprising a proportion of functioning matter having maintained function may be cultivated after loading to allow functioning matter to replicate or otherwise increase in content. It is a significant feature of the invention that functioning is at least in part preserved, and this is distinct from processes in which all functioning is destroyed, ie, processes which are inherently prejudicial to functioning.

Factors affecting the proportion of functioning matter that retains function may be varied and include the selected plasticising conditions, the fluid(s) and the functioning matter. It will be appreciated that functioning matter as hereinbefore defined comprises a plurality of independent entities of matter, some of which may be more resilient than others and some of which may be exposed to harsher conditions than others, eg local fluctuations or variations in temperature and the like. Preferably a proportion of functioning matter which does not freeze or refreeze is at least 20%, preferably at least 40%, more preferably at least 60% up to substantially 100% of functioning matter.

The product composite may be assessed for preserved functioning, after contact with plasticising fluid loading into polymer. Preferably viability of cells is assessed by light microscopy, or by using an Alamar Blue assay. The Alamar Blue assay measures cell proliferation, hence viability, and is a REDOX reaction which produces a fluorescent product upon reduction of the reagent by the cells. Fluorescence therefore correlates directly with cell viability.

Preferably in the case that the functioning matter requires nutrients or the like, for example comprises biological cells, the process includes a further step of maintaining the product composite in culture, or under dormant or suspended state conditions prior to culturing. Maintaining the composite in culture comprises providing all cell nutrients required for the survival of the functioning matter and removing any effluent produced, and more preferably comprises providing all nutrients required for proliferation and growth of the functioning matter and removing effluent produced. Preferably maintaining the composite in culture comprises contacting with a source of oxygen, water, carbohydrate and essential minerals, typically in the form of cell culture media as known in the art. The composite may be immersed in culture or may be drip fed, or otherwise contacted with culture media as known in the art. Other techniques encouraging cell growth and fusion, such as cyclic mechanical straining, applied electric field and the like may be employed.

Suitably maintaining the composite under dormant or suspended state conditions comprises maintaining under the prevailing or different preservation conditions employed heretofor during contact with plasticisation fluid.

The polymer is suitably in the solid phase or is a highly viscous, viscous or non-viscous fluid and may present limited or good mixing characteristics. Solid phase polymer may be particulate, e.g. in the form of granules, pellets, microspheres, powder, or monolithic e.g. matrix form. Plasticising conditions comprise conditions of reduced viscosity to plasticise and/or swell the polymer. It is known that particulate polymer agglomerates on plasticisation to a larger structure. This may revert to a particulate composite or form a monolithic composite on release of plasticising fluid, as hereinbelow defined.

Reference herein to a plasticising fluid is to a fluid which is able to plasticise polymer in its natural state or in supercritical, near critical, dense-phase or subcritical state. Fluid may be liquid or gaseous, and is preferably selected for a suitable density which is capable of plasticising a given polymer, fluid density may be in the range 0.001 g/ml up to 10 g/ml for example 0.001 g/ml up to 2 g/ml.

Plasticising conditions comprises elevated or ambient or reduced temperature, and/or elevated or ambient pressure as hereinbefore defined. Fluid may be selected for effective plasticisation of a given polymer under conditions which are amenable to the functioning matter or alternatively fluid is selected by preferred chemical type and suitable plasticising conditions are chosen for that fluid, preferably selecting a first amenable condition (T) and compensating with second condition (P) to obtain desired density.

Plasticising conditions typically comprises a temperature less than, equal to or greater than the fluids critical temperature (Tc) in the range -200°C to +500°C, for example -200°C to 200°C. Preferably plasticising conditions according to the present invention comprise a temperature in the range -100 to +500 °C, more preferably -100 to +100°C, preferably -20 to +500 °C or -20 to +80°C. Processing at temperatures in excess of -2 °C is typically more convenient in practical terms than processing at lower temperatures but there may be advantages in operating in a temperature range of less than +2 °C, eg -200 to +2 °C. In the case of chemically or physically preserved functioning matter a preferred temperature range such as to prevent (re)freezing during processing, for example using carbon dioxide as plasticising fluid, may be less than substantially +4°C, for example substantially -100 to +4°C, more preferably -20 to +4°C or is greater than substantially 35°C for example substantially 35 to 65°C, more preferably 35 to 55°C or 45 to 60°C. In the case of functioning matter not preserved in dry form but by imposing pressure constraint, a preferred temperature range is less than substantially 37°C, preferably substantially +4 to + 37 °C, for example 10 to 37°C, preferably 10 or 15 to 35 °C, more preferably 20 to 33°C, most preferably approximately 28 to 33°C (CO₂).
Optimum temperature in these ranges may depend in part on the nature of functioning matter to be processed and on the plasticising fluid or fluids to be employed, as hereinbefore described. For most fluids a typical temperature may be in the range approximately 10 to 15°C, to 25°C, 25 to 30°C, 30 to 35°C, 35 to 45°C or 45 to 55°C. Other sub ranges may be envisaged and are within the scope of the invention. Preferably the lowest temperature is employed which is compatible with sufficient lowering of the polymer Tg to achieve plasticisation. To operate at ambient temperature, the process of the invention may require compensation by increase in pressure.

Throughout contact with plasticising fluid, under conditions which typically comprise ambient temperature or elevated temperature, frozen functioning matter is preferably maintained frozen, for example by conducting the process in a vessel and cooling the vessel; or is allowed to thaw during processing, which also maintains viability. Significantly the process of the invention ensures frozen functioning matter is not allowed to thaw and refreeze during processing which can prejudice viability.
Plasticising fluid typically comprises a pressure less than, equal to or greater than the plasticising fluids critical pressure (Pc) from in excess of 1 x 10⁵ Nm⁻² to 1 x 10⁹ Nm⁻² (1 bar to 10000 bar), preferably 1 x 10⁵ Nm⁻² to 1 x 10⁸ Nm⁻² for example 2x10⁵ Nm⁻² to 8x10⁷ Nm⁻² (1 to 1000 bar, for example 2 to 800 bar). Preferably plasticising conditions according to the present invention comprise a pressure in the range 2 x 10⁵ Nm⁻² to 4 x 10⁷ Nm⁻² (2 to 400 bar), more preferably 5 x 10⁵ Nm⁻² to 2.65 x 10⁷ Nm⁻² (5 to 265 bar), most preferably 5 x 10⁵ Nm⁻² to 75 x 10⁵ Nm⁻² (5 to 75 bar). In the case of functioning matter preserved by imposing pressure constraint we have surprisingly found that operation at a pressure in a preferred range-of 5 x 10⁵ Nm⁻²to 75 x10⁵ Nm⁻² (5 to 75 bar) and at temperatures in the range of +4 to +37 °C maintains viability of functioning matter.

Optimum pressure in these ranges may depend in part on the nature of functioning matter to be processed and on the plasticising fluid or fluids to be employed, as hereinbefore described. For most fluids pressure will be in the range approximately 3 x 10⁶ Nm⁻² to 4 x 10⁶ Nm⁻² (30 to 40 bar), 4 x 10⁶Nm⁻² to 5 x 10⁶ Nm⁻² (40 to 50 bar), 5 x 10⁶ Nm⁻² to 6 x 10⁶ Nm⁻² (50 to 60 bar), 6 x 10⁶ Nm⁻² to 7.5 x 10⁶Nm⁻² (60 to 75 bar), most preferably approximately 3.4 x 10⁶ Nm⁻² to 7.5 x 10⁶ Nm⁻² (34 to 75 bar) (dense phase or supercritical CO₂). Other sub ranges may be envisaged and are within the scope of this invention.
Fluid may be provided at plasticising conditions prior to contacting with polymer and functioning matter or may be brought to plasticising conditions in contact with one or both of polymer and functioning matter. Swelling and plasticisation may be simultaneous or sequential or plasticisation may occur without swelling.
The process is conducted for a suitable contact time of plasticising fluid and functioning matter which can be employed without prejudicing functioning of matter. For example it is important that the process is conducted with contact time such that there is little or no thawing of frozen functioning matter. In a particular advantage the process may be carried out for very short contact time of plasticising fluid and functioning matter of 2 milliseconds up to 10 minutes, more preferably 20 milliseconds to 5 minutes, more preferably 1 second to 1 minute, more preferably 2 to 30 seconds, most preferably 2 to 15 seconds. In this case a non-uniform distribution may be acceptable. Alternatively contact time of plasticising fluid and functioning matter may be up to 2 hours, for example 10 minutes to 2 hours or 10 minutes to 1 hour, in the case of more resilient functioning matter or certain plasticising fluid(s), or where extended processing time is required to form the composite, for example by virtue of chosen plasticising conditions.

Contact time of plasticising fluid and polymer may be up to 5 hours.

Preferably the polymer and functioning matter are immersed in or contacted with the plasticising fluid in a batchwise process. The components of the polymer composite may be combined in any desired order, prior to, or during application of plasticising conditions. Contacting may be of the components simultaneously or sequentially, for example first contacting polymer and functioning matter and subsequently introducing plasticising fluid, or preferably first contacting polymer and plasticising fluid and subsequently introducing functioning matter.

In a first embodiment plasticisation of polymer is conducted initially, and subsequently an amount of functioning matter is introduced for a period sufficient to combine with the polymer. Preferably, in this embodiment, preserved functioning matter is loaded into a (cooled) chamber, polymer is loaded into a main chamber and plasticised in contact with plasticising fluid, preserved functioning matter is discharged from cooled to main chamber with rapid contacting and release of plasticising fluid

In an alternative embodiment the polymer is partially plasticised before contact with functioning matter and completing plasticisation.

In an alternative embodiment the polymer and functioning matter are contacted simultaneously with plasticising fluid. In this case functioning matter may be admixed with polymer *in situ* or may be supported on or admixed with polymer prior to plasticising.

In the case of physically preserving by cryopreservation the functioning matter may be frozen separately or in supported or admixed form, supported on or admixed with polymer. For example the polymer may be partially plasticised before contacting with functioning matter, and the combined functioning matter and polymer then frozen prior to further exposure to plasticising conditions as hereinbefore defined.

Contact time of preserved functioning matter and plasticising fluid may be critical. Contact time of polymer and plasticising fluid may be dictated in part by polymer MW, Tg, and/or mass of polymer, whereby contact is sufficient for plasticisation.

Pressurising plasticising fluid may be *in situ,* or ex situ prior to contacting with functioning matter and/or polymer as hereinbefore defined. The pressurisation period whereby in the case of *in situ* or ex situ pressurisation the fluid is pressurised or is introduced to the functioning matter and polymer, is suitably for a period of 1 second to 3 minutes, more preferably from 1 second to 1 minute, more preferably from 1 to 45 seconds.

The process may be carried out with or without stirring or blending. Blending and conditions may be selected to assist plasticisation or according to the desired uniformity and distribution of loading. In the case that uniform distribution is required the process preferably comprises blending for prolonged period and/or high intensity. In the case that non-uniform distribution is envisaged, the process may be carried out simply with stirring. Preferably the process is carried out under conditions of stirring to combine the functioning matter and polymer, for purpose of loading but without need for uniform distribution.

Blending may be by physical mixing, pumping, agitation for example with aeration or fluidising gas flow, lamellar flow or otherwise impregnation or diffusion of plasticising fluid throughout the polymer and functioning matter. Stirring is typically with use of stirrers and impellers, preferably helical impellers such as helical ribbon impellers for enhanced blending and the like.

Blending may be for a period of 1 millisecond to 5 hours and maybe for the duration of contacting with plasticising fluid or otherwise. Preferably blending is for the duration of contacting with plasticising fluid for a period corresponding to the period for fluid contacting as hereinbefore defined.

The process comprises subsequently releasing the plasticising fluid. In the case that plasticising conditions comprises elevated pressure, release is under reduced pressure conditions, conducted over a desired depressurisation period, whereby the polymer composite is isolated loaded with functioning matter. Depressurisation may be achieved *in situ,* by depressurising a pressure vessel in which the process is carried out, whereby a monolithic block of polymer composite is obtained. Alternatively the contents of a pressure vessel in which the process is conducted may be discharged into a second pressure vessel at lower pressure whereby a homogeneous powder of polymer composite as hereinbefore defined is obtained by known means.

Depressurisation period may be selected-to foam the polymer if desired, and therefore determines the porosity of composite. Transition is preferably rapid over a period of from 1 ms to 10 minutes, preferably from 1 second to 3 minutes, more preferably from 1 second to 1 minute, more preferably from 1 to 45 seconds. Transition may be from 1 to 3 seconds for high porosity polymer. Alternatively plasticising fluid may be released in manner to allow fluid diffusion out of the polymer, avoiding foaming, to create a non-porous structure. Typically this is achieved by prolonged gradual release of fluid over a period of in excess of 10 minutes up to 12 hours. Preferably transition is to near ambient pressure i.e. substantially 1 atm (101.325 kPa).

Pressure release may confer further cooling, maintaining the preserved state of frozen functioning matter.

The process may be carried out in the presence or absence of functioning matter compatible carriers or agents such as preservation agents. In a particular advantage the process may be carried out in the presence of solvent carriers and like which might otherwise damage the functioning matter in non-preserved state. Suitable preservation agents include water, to prevent non intentional desiccation of hydrated functioning matter, cryopreservation agents which prevent ice crystal formation such as DMSO, trehalose, dextran and the like.

A plasticising fluid as hereinbefore defined may comprise any fluid which is capable of plasticising a desired polymer. As is known in the art such fluids may be subjected to conditions of elevated temperature and pressure increasing density thereof up to and beyond a critical point at which the equilibrium line between liquid and vapour regions disappears. Supercritical fluids are characterised by properties which are both gas like and liquid like. In particular, the fluid density and solubility properties resemble those of liquids, whilst the viscosity, surface tension and fluid diffusion rate in any medium resemble those of a gas, giving gas like penetration of the medium

Preferred plasticising fluids include carbon dioxide, di-nitrogen oxide, carbon disulphide, aliphatic C₂₋₁₀ hydrocarbons such as ethane, propane, butane, pentane, hexane, ethylene, and halogenated derivatives thereof such as for example carbon tetrafluoride or chloride and carbon monochloride trifluoride, and fluoroform or chloroform, C₆₋₁₀ aromatics such as benzene, toluene and xylene, C₁₋₃ alcohols such as methanol and ethanol, sulphur halides such as sulphur hexafluoride, ammonia, xenon, krypton and the like, or a mixture thereof. Typically these fluids may be brought into plasticising conditions at temperature of between -200°C to + 500°C and pressures of in excess of 1 x 10⁵ Nm⁻² to 1 x 10⁹ Nm⁻² (1 bar to 10000 bar), as hereinbefore defined. It will be appreciated that the choice of fluid may be made according to its properties, for example diffusion and polymer plasticisation. Preferably the fluid acts as solvent for residual components of a polymer composite as hereinbefore defined but not for polymer or functioning matter as hereinbefore defined, Choice of fluid may also be made with regard to critical conditions which facilitate the commercial preparation of the polymer as hereinbefore defined. Supercritical conditions of some fluids are shown in Table 1.

**Table 1**

| Fluid | Critical Temperature /°C | Critical Pressure / x 10⁵ Nm⁻²(bar) |
|---|---|---|
| Carbon dioxide | 31.1 | 73.8 |
| Ethane | 32.4 | 48.1 |
| Ethylene | 9.3 | 49.7 |
| Nitrous oxide | 36.6 | 71.4 |
| Xenon | 16.7 | 57.6 |
| Fluoroform CHF₃ | 26.3 | 48.0 |
| Monofluoromethane | 42 | 55.3 |
| Tetrafluoroethane | 55 | 40.6 |
| Sulphur hexafluoride | 45.7 | 37.1 |
| Chlorofluoromethane | 29 | 38.2 |
| Chlorotrifluoromethane | 28.9 | 38.7 |
| Nitrogen | -147 | 33.9 |
| | | |
| Ammonia | 132.5 | 111.3 |
| Cyclohexane | 280.3 | 40.2 |
| Benzene | 289.0 | 48.3 |
| Toluene | 318.6 | 40.6 |
| Trichlorofluoromethane | 198.1 | 43.5 |
| Propane | 96.7 | 41.9 |
| Propylene | 91.9 | 45.6 |
| Isopropanol | 235.2 | 47.0 |

Preferably the plasticising fluid comprises carbon dioxide optionally in admixture with any further fluids as hereinbefore defined or mixed with conventional solvents, so-called "modifiers". CO₂ is generally approved by regulatory bodies for medical-applications, is chemically inert, leaves no residue and is freely available.

The plasticising fluid may be present in any effective amount with respect to the polymer. Preferably the plasticising fluid is provided at a desired concentration in the reaction vessel to give a desired plasticisation and/or swelling of polymer. Such range may be from 1% to 200% of the polymer weight, e.g. with plasticising fluid in sufficient excess to achieve 10% to 40% absorption with respect to polymer weight.

The functioning matter may be present in any effective amount with respect to polymer. Proliferative functioning matter may be provided in the process at a desired starting concentration allowing for survival and post processing growth. Typical values are therefore 1x10⁻¹² wt% to 99.9 wt%, for example 1x10⁻⁹ wt% to 99.9 wt%, preferably 0.01 to 99.9 wt%, more preferably 0.1 to 99.0 wt%, more preferably greater than 0.5 wt% or 1.0 wt% up to 50 wt%.

Biological functioning matter is typically any nutrient dependent, biological matter which is characterised by replication, division, regeneration, growth, proliferation or the like, and which is harmed by contact with plasticising fluid under conventional plasticising conditions.

Biological functioning matter is preferably selected from any subcellular, cellular or multicellular matter and aggregates and mixtures thereof. Preferably functioning matter is selected from mammalian, plant and bacterial cells including (subcellular) organelles and aggregates thereof including pancreatic islet or liver spheroids and the like; non cellular matter such as liposomes optionally as carrier of matter such as protein or enzymes which become sensitive to dense phase fluid in presence of liposomic water. Cellular matter is more preferably selected from mammalian and plant prokaryotic and eukaryotic cells and mixtures and aggregates thereof, most preferably mammalian cells selected from fibroblasts, fibrochondrocytes, chondrocytes, bone forming cells such as osteoblasts and osteoclasts, bone marrow cells, hepatocytes, cardiomycytes, blood vessel forming cells, neurons, myoblasts, macrophages, microvascular endothelium cells and mixtures thereof and collagen. Biological functioning matter may be naturally occurring or synthetic, for example cells may be genetically modified or mutated in known manner to incorporate, delete or modify components.

Preferably functioning matter is selected from a component, or precursor, derivative or analogue thereof, of a host structure into which implantation or incorporation is desired and preferably comprises matter intended for growth or repair, shielding, protection, modification or modelling of a human, animal, plant or other living host structure for example the skeleton, organs, dental structure and the like; to combat antagonists; for metabolism of poisons, toxins, waste and the like or for synthesis of useful products by natural processes, for bioremediation, biosynthesis, biocatalysis or the like.

In a particular advantage the process of the invention enables instant production of functioning matter laden scaffolds, for example cell laden scaffolds, in one step, in contrast with current practice of forming a scaffold and seeding with cells over a 24 or 48 hour period. This may have particular advantages in the delivery for example of stem and progenitor cells to patients.

In a further advantage we believe that the processing may confer a degree of sterilisation by the plasticising fluid, whereby it selectively inactivates non preserved matter, such as bacteria present in the atmosphere and the like.

The functioning matter may be in any desired form suited for the intended application, for example in solid, semi-solid such as thixotrope or gel form, semi-fluid or fluid such as paste or liquid form, and may be miscible or immiscible but is insoluble in the polymer and dense phase fluid, eg as a suspension. It may be convenient to adapt the functioning matter form to render it in preferred form for processing and the intended application. The matter is preferably in the form of solid particles having particle size selected according to the intended application. Preferably particle size is of similar or lesser order to that of the polymer composite particle size, and of any pores, preferably 10⁹m - 10⁻²m, for example of the order of nanometers, micrometers, millimetres or centimetres, more preferably 50 micron to 1 centimetre for cells and aggregates.

The polymer composite may additionally comprise any additional biofunctional components as disclosed in WO 98/51347, including but not limited to the following examples typically classed as living matter; (pharmaceutical) drugs and veterinary products; agrochemicals as pest and plant growth control agents; human and animal health products; human and animal growth promoting, structural, or cosmetic products including products intended for growth or repair or modelling of the skeleton, organs, dental structure and the like; absorbent biofunctional materials for poisons, toxins and the like.

Pharmaceuticals and veterinary products, i.e. drugs, may be defined as any pharmacologically active compounds that alter physiological processes with the aim of treating, preventing, curing, mitigating or diagnosing a disease.

Drugs may be composed of inorganic or organic molecules, peptides, proteins, enzymes, oligosaccharides, carbohydrates, nucleic acids and the like.

Drugs may include but not be limited to compounds acting to treat the following:
Infections such as antiviral drugs, antibacterial drugs, antifungal drugs, antiprotozal drugs, anthelmintics,
Cardiovascular system such as positive inotropic drugs, diuretics, antiarrhythmic drugs, beta-adrenoceptor blocking drugs, calcium channel blockers, sympathomimetics, anticoagulants, antiplatelet drugs, fibrinolytic drugs, lipid-lowering drugs;
Gastro-intestinal system agents such as antacids, antispasmodics, ulcer-healing, drugs, anti-diarrhoeal drugs, laxatives, central nervous system, hypnotics and anxiolytics, antipsychotics, antidepressants, central nervous system stimulants, appetite suppressants, drugs used to treat nausea and vomiting, analgesics, antiepileptics, drugs used in parkinsonism, drugs used in substance dependence; Malignant disease and immunosuppresion agents such as cytotoxic drugs, immune response modulators, sex hormones and antagonists of malignant diseases;
Respiratory system agents such as bronchodilators, corticosteroids, cromoglycate and related therapy, antihistamines, respiratory stimulants, pulmonary surfactants, systemic nasal decongestants;
Musculoskeletal and joint diseases agents such as drugs used in rheumatic diseases, drugs used in neuromuscular disorders; and
Immunological products and vaccines.

Agrochemicals and crop protection products may be defined as any pest or plant growth control agents, plant disease control agents, soil improvement agents and the like. For example pest growth control agents include insecticides, miticides, rodenticides, molluscicides, slugicides, vermicides (nematodes, anthelmintics), soil fumigants, pest repellants and attractants such as pheromones etc, chemical warfare agents, and biological control agents such as microorganisms, predators and natural products;
plant growth control agents include herbicides, weedicides, defoliants, desiccants, fruit drop and set controllers, rooting compounds, sprouting inhibitors, growth stimulants and retardants, moss and lichen controllers and plant genetic controllers or agents;
plant disease control agents include fungicides, viricides, timber preservatives and bactericides; and
soil improvement agents include fertilisers, trace metal additives, bacterial action control stimulants and soil consolidation agents.

Human and animal health or growth promoting products may be defined as any of the above intended for general health purpose, including vitamins, nutrients, steroids, and the like.

Preferred biofunctional components include growth promoters, biocompatibilisers, vitamins, proteins, glycoproteins, enzymes, nucleic acid, carbohydrates, minerals, nutrients, steroids, ceramics and the like; and may include living matter such as spores, viruses, bacteria and the like. In particular growth factors such as basic Fibroblastic Growth Factor, acid Fibroblastic Growth Factor, Epidermal Growth Factor, Human Growth Factor, Insulin Like Growth Factor, Platelet Derived Growth Factor, Nerve Growth Factor and Transforming Growth Factor; antitumorals such as BCNU or 1, 3-bis (2-chloroethyl) -1-nitrosourea, daunorubicin, doxorubicin, epirubicin, idarubicin, 4-demethoxydaunorubicin 3'-desamine-3' - (3-cyano-4-morpholinyl) -doxorubicin, 4-demethoxydaunorubicin-3' -desamine-3' - (2-methoxy-4-morpholinyl) -doxorubicin, etoposide and teniposide; hormones such as LHRH and LHRH analogues; and steroideals for birth control and/or antitumoral action such as medroxyprogesterone acetate or megestrol acetate, tricalcium phosphate or the class of apatite derivatives, for example calcium hydroxyapatite which functions as a bone or dental component and promotes biocompatability, silicon which functions as a tissue modelling component, and analogues, precursors or functioning derivatives thereof, bioactive species such as collagen, bioglasses and bioceramics, other minerals, hyaluran, polyethyleneoxide, CMC (carboxymethylcellulose), proteins, organic polymers, and the like and components adapted for incorporation as implants into meniscus, cartilage, tissue and the like and preferably promote growth, modelling, enhancing or reinforcing of collagen, fibroblasts and other natural components of these host structures.

Additional biofunctional component(s) may be mixed with the polymer and functioning matter or may be introduced by subsequent soaking or impregnation of functioning matter laden product composite.

Biofunctional components may be present in any desired amount for example as hereinbefore defined for functioning matter. For example a composite may comprise 80 wt% hydroxyapatite, 10 wt% cells, less than 1 wt% growth factor and more than 1 wt% antibiotic.

Accordingly in a preferred embodiment there is provided according to the present invention a process as hereinbefore defined for the preparation of a polymer composite comprising biofunctional matter as hereinbefore defined and loaded with functioning matter as hereinbefore defined wherein the process comprises in a first stage contacting the biofunctional material and polymer and a plasticising fluid as hereinbefore defined under plasticising conditions as hereinbefore defined to plasticise and/or swell the polymer and incorporate the biofunctional material and subsequently introducing an amount of functioning matter and combining with polymer, and releasing the plasticising fluid to obtain the polymer composite, wherein contacting is at a pressure in the range 1 x 10⁵ Nm⁻² to 1 x 10⁸ Nm⁻² (1 to 1000 bar) and a temperature in the range -200 to +200°C selected in manner that at least an amount of functioning matter does not freeze or refreeze during processing or if at a temperature at which freezing or refreezing may occur, that either matter is desiccated or a pressure constraint is applied in a range having a maximum pressure less than 1 x 10⁸ Nm-² (1000 bar) throughout contact of functioning matter and plasticising fluid, whereby at least an amount of functioning matter retains its function in the polymer composite. Or example we have observed that freezing or refreezing may take place during processing with carbon dioxide as plasticising fluid at least in the temperature range +4 to +35°C. Functioning matter may be chemically or physically preserved against harmful effects associated with the plasticising fluid as hereinbefore defined and is maintained in preserved state preferably in dry form, for example may be cryopreserved or desiccated throughout contact therewith. Alternatively matter may be in hydrated, non frozen form.

If porous, a composite may comprise open or closed cell pores. Composite obtained with a very open porous structure, known as miarooellular, is ideal for biomedical and biocatalytic applications for example supporting growth of blood vessels and collagen fibres throughout the matrix, and forming structures resembling bone, meniscus, cartilage, tissue and the like, and providing a structure for throughput of substrate for biocatalysis and bioremediation and the like.

Non-porous, open or closed cell composite may be useful for biodegradable staged or prolonged release delivery applications of functioning matter or biofunctional material not requiring leaching in or out or other access. Release may be *in vitro* or *in vivo* and by parenteral, oral, intravenous, application or surgical for release proximal to the treatment locus, e.g. in tissue tumor treatment, or hyperthermic bone tumor treatment.

A porous polymer composite may be obtained with uniform or varied porosity, preferably provides pores of at least two different orders of magnitude, for example of micro and macro type, each present in an amount of between 1 and 99% of the total void fraction of the polymer composite.

Reference herein to micro and macro pores is therefore to be understood to be respectively pores of any unit dimension and its corresponding 10ⁿ multiple. For example micro pores may be of the order of 10⁻⁽¹⁰⁻⁷⁾m with respective macro pores of the order of 10⁻⁽⁷⁻⁵⁾m, preferably 10⁻⁽⁸⁻⁷⁾m and 10⁻⁽⁶⁻⁵⁾m respectively, more preferably of micron and 10² micron order. The pores may be of any desired configuration. Preferably the pores form a network of tortuous interlinking channels, more preferably wherein the micro pores interlink between the macro pores.

Functioning matter may be distributed throughout relatively smaller and relatively larger pores or confined to larger pores. Functioning matter may be embedded in the walls of pores or may be freely supported but not encased in polymer matrix.

Macro pores are suitably of magnitude and distribution whereby embedding with functioning matter to a desired thickness creates release loci into which functioning matter may proliferate and configure in naturally occurring manner. Micropores create a channel structure throughout the polymer composite, which provides an open cell structure for supply and removal of materials, in particular nutrient and effluent, and for concomitant proliferation and spread of cells throughout the polymer. Different particle size functioning matter may selectively distribute between smaller and larger pores. Other directing means may be employed to direct anisotropic cells (eg nerve, muscle, blood vessel cells) to channels formed by smaller pores, and direct isotropic cells (eg tissue, bone, cornea, marrow) to larger pores.

The process may be controlled in manner to determine the dimensions and void fraction of micro and macro pores and the morphology of the final product. The period for dense phase fluid release determines in part the level of porosity. Additionally the difference in pressure is proportional to porosity. Also a higher critical temperature confers a higher porosity. The composite is suitably obtained with porosity of 15% to 75% or greater, preferably 50% up to 97%.

Suitably the polymer retains its solid or highly viscous fluid form subsequent to release of plasticising fluid, in order to retain the porous structure induced by the fluid.

Further processing of the polymer, for example additional extraction with super critical fluid as known in the art or with other extractants, post-polymerisation and cross-linking, may be subsequently performed as required and as known in the art.

The polymer may be selected from any known polymer, (block) copolymer, mixtures and blends thereof which may be crosslinked or otherwise which is suited for introduction into or association with the human or animal body, plants or other living matter, or *in vitro,* or for use in the environment in non-toxic manner. Suitable polymer materials are selected from synthetic biodegradable polymers as disclosed in "Polymeric Biomaterials" ed. Severian Dumitriu, ISBN 0-8247-8969-5, Publ. Marcel Dekker, New York, USA, 1994, bioresorbable polymers synthetic non-biodegradable polymers; and natural polymers.

Preferably the polymer is selected from homopolymers, block and random copolymers, polymeric blends and composites of monomers which may be straight chain, (hyper) branched or cross-linked.

Polymer may be of any molecular weight for the desired application, and is suitably in the range of from 1 to 1,000,000 repeat units. Higher molecular weight may be useful for longer release patterns or slower degradation.

Polymers may include but are not limited to the following which are given as illustration only.

Synthetic biodegradable polymers may be selected from:
Polyesters including poly(lactic acid), poly(glycolic acid), copolymers of lactic and glycolic acid, copolymers of lactic and glycolic acid with poly(ethylene glycol), poly(e-caprolactone), poly(3-hydroxybutyrate), poly(p-dioxanone), poly(propylene fumarate);
Preferably polylactides include DD, DL, LL enantiomers and are prepared from D and L lactic acid and glycolic acid monomers, or a combination thereof, or monomers such as 3-propiolactone tetramethylglycolide, b-butyrolactone, 4-butyrolactone, pivavolactone and intermolecular cyclic esters of alpha-hydroxy butyric acid, alpha-hydroxyisobutyric acid, alpha-hydroxyvaleric acid, alpha-hydroxyisovaleric acid, alpha-hydroxycaproic acid, alpha-hydroxy- alpha-ethylbutyric acid, alpha-hydroxyisocaproic acid, alpha-hydroxy-3-methylvaleric acid, alpha-hydroxyheptanoic acid, alpha-hydroxyoctanoic acid, alpha-hydroxydecanoic acid, alpha-hydroxymyristic acid, alpha-hydroxystearic acid, and alpha-hydroxylignoceric acid. It is most preferred to use lactic acid as sole inonomer or lactic acid as the principal monomer with glycolic acid as the comonomer. The latter are termed poly(lactide-co-glycolide) copolymers; particularly suitable are polymers prepared from lactic acid alone, glycolic acid alone, or lactic acid and glycolic acid wherein the glycolic acid is present as a comonomer in a molar ratio of 100:0 to 40:60;
Poly (ortho esters) including Polyol/diketene' acetals addition polymers as described by Heller in: ACS Symposium Series 567, 292-305, 1994;
Polyanhydrides including poly(sebacic anhydride) (PSA), poly(carboxybisbarboxyphenoxyphenoxyhexane) (PCPP), poly[bis(p-carboxyphenoxy) methane] (PCPM), copolymers of SA, CPP and CPM, as described by Tamada and Langer in Journal of Biomaterials Science- Polymer Edition, 3, 315-353,1992 and by Domb in Chapter 8 of the Handbook of Biodegradable Polymers, ed. Domb A.J. and Wiseman R.M., Harwood Academic Publishers;
Poly(amino acids); polyacetals; polyketals; polyorthoesters;
Poly(pseudo amino acids) including those described by James and Kohn in pages 389-403 of Controlled Drug Delivery Challenges and Strategies, American Chemical Society, Washington DC.;
Polyphosphazenes including derivatives of poly[(dichloro) phosphazene], poly[(organo) phosphazenes], polymers described by Schacht in Biotechnology and Bioengineering, 52, 102-108, 1996; and
Azo polymers
   Including those described by Lloyd in International Journal of Pharmaceutics, 106, 255-260, 1994.

Synthetic Non-biodegradable Polymers may be selected from:
Vinyl polymers including polyethylene, poly(ethylene-co-vinyl acetate), polypropylene, poly(vinyl chloride), poly(vinyl acetate), poly(vinyl alcohol) and copolymers of vinyl alcohol and vinyl acetate, poly(acrylic acid) poly(methacrylic acid), polyacrylamides, polymethacrylamides, polyacrylates, Poly(ethylene glycol), Poly(dimethyl siloxane), Polyurethanes, Polycarbonates, Polystyrene and derivatives.

Natural Polymers may be selected from carbohydrates, polypeptides and proteins including:
Starch, Cellulose and derivatives including ethylcellulose, methylcellulose, ethylhydroxyethylcellulose, sodium carboxymethylcellulose; Collagen; Gelatin; Dextran and derivatives; Alginates; Chitin; and Chitosan;
Preferably a non biodegradable polymer is selected from polymers such as ester urethanes or epoxy, bis-maleimides, methacrylates such as methyl or glycidyl methacrylate, tri-methylene carbonate, di-methylene tri-methylene carbonate; biodegradable synthetic polymers such as glycolic acid, glycolide, lactic acid, lactide, p-dioxanone, dioxepanone, alkylene oxalates and caprolactones such as gamma-caprolactone.

Polymer substrate may be obtained from its precursors in the process of the invention. The precursors may react to form the polymer substrate(s) *in situ* during or subsequent to dense phase fluid processing.

The polymer may comprise any additional polymeric components having performance enhancing or controlling effect, for example determining the degree and nature of cross-linking for improved permeability by bodily fluids or pharmaceutically effective agent, flexural and general mechanical properties, electrical properties and the like.

Additional components which may be incorporated during the manufacture of the polymer composite, for example initiators, accelerators, hardeners, stabilisers, antioxidants, adhesion promoters, fillers and the like may be incorporated within the polymer.

If it is desired to introduce an adhesion promoter into the polymer composite, the promoter may be used to impregnate or coat particles of functioning matter prior to introduction into the polymer composite, by means of simple mixing, spraying or other known coating steps, in the presence or absence of fluid as hereinbefore defined. Preferably coating is performed in conjunction with mixing with fluid as hereinbefore defined whereby excellent coating is obtained. For example the adhesion promoter is dissolved in fluid as hereinbefore defined and the solution is contacted with functioning matter particles as hereinbefore defined. Alternatively the adhesion promoter is introduced into the autoclave during the mixing and/or polymerisation step whereby it attaches to the functioning matter particles in desired manner.

Preferably the total amount of fillers including the functioning matter lies in the region of 0.01-99.9 wt %, preferably 0.1-99 wt%.

In a further aspect of the invention there is provided a polymer composite obtained with the process of the invention as hereinbefore defined, for example comprising a polymer loaded with functioning matter by the process of the invention whereby functioning matter has been in non-preserved or preserved state or has been preserved by imposing pressure constraint throughout the process.

In a further aspect of the invention there is provided a polymer composite comprising polymer loaded with functioning matter as hereinbefore defined which is non-established. Reference herein to non-established matter is to instantly loaded functioning matter, and to established matter is to matter which is loaded and proliferated, grown, adhered or otherwise modified post loading. In the case that matter has an absolute need for nutrients, the composite is rendered in suspended state pending supply or is cultured in contact with a nutrient supply with optional effluent removal in manner to enable establishment of functioning matter.

Established cellular matter is suitably present as plaques, rods or other extended structure types, in particular for cellular matter, with cell fusion. Cellular matter may diversify after culturing to provide different cell types.

The composite of the invention may be distinguished from prior art composite prepared by simple impregnation techniques which either comprise functioning matter at the surface only, or which comprise internally proliferated matter, which is established and can be distinguished in form from the non-established loaded matter of the invention.

The polymer composite may be in desired form suitable for the hereinbefore mentioned applications. Suitably the composite may be obtained in granular or monolith form and is preferably in monolith form for use as a scaffold or drug delivery device.

For use as bioremediation, biocatalyst or biobarrier for human or animal or plant matter, the composite may be in a suitable shaped form or may be impregnated into a shaped product, to provide a barrier film, membrane, layer, clothing or sheet.

For use as a structural component, for example comprising the polymer and optional additional synthetic or natural metal, plastic, carbon or glass fibre mesh, scrim, rod or like reinforcing for medical or surgical insertion, the composite may be adapted for dry or wet insertion into a desired host structure, for example may be in powder, pellet, granule or monolith form suited for insertion as a solid monolith into bone or tissue, as fillers or cements for wet insertion into bone or teeth or as solid aggregates or monoliths for orthopaedic implants such as pins, or dental implants such as crowns etc. Insertion may be by injection, surgical insertion and the like.

The polymer composite may be of any desired particle size in the range of 0.1 or 1 micron powders, preferably from 50 micron or 200 micron for cellular loading up to monoliths of the order of 20 centimetres magnitude. It is a particular advantage of the present invention that the polymer composite is obtained in the desired form in uniform size particles such as powder, pellets and the like. Accordingly if it is desired to obtain a random or discrete distribution of particle size the polymer composite may be milled or may be blended from different size batches.

Composite particle size may be controlled according to known techniques by controlled removal of plasticising fluid. If it is desired to obtain particulate composite, the process mixture is suitably removed from the mixing chamber under plasticising conditions into a separate container under ambient conditions through a nozzle or like orifice of desired aperture, and under desired difference of conditions and removal rate, adapted to provide the desired particle size. Spray drying apparatus and techniques may commonly be employed for the technique.

If it is desired to obtain a polymer composite in the form of monoliths, the plasticising fluid is suitably removed using known techniques for foaming polymers. Accordingly the polymer mix is retained in the reaction vessel and conditions are changed from plasticising to ambient at a desired rate to cause removal of the fluid from the polymer mix. Depending on the nature of the polymer it is possible to obtain the monolith in porous foamed state, if desired, having interconnecting pores and channels created by the removal of the plasticising fluid, simply by selecting a polymer consistency which is adapted to retain its foamed state.

Monoliths may be formed into desired shape during the processing thereof, for example by removal of plasticising fluid from a mixing vessel, or from a mould internal to mixing vessel, having the desired monolith shape. Alternatively monolith may be removed from the mixing vessel and cut to desired shape or transferred directly into a mould.

In a further aspect of the invention there is provided a scaffold comprising a polymer composite loaded with functioning matter, optionally additionally comprising biofunctional materials, as hereinbefore defined, suitably sized and shaped for a desired application as hereinbefore defined. The scaffold is suitably provided in suspended state as hereinbefore defined, and may have been cultured subsequent to composite preparation. Preferably the scaffold is frozen, and is in non-established state or established state.

A scaffold according to the invention is suitably in the form of a surgical implant, synthetic bone composite, an organ module, etc. or biocatalyst or biobarrier for synthesis or remediation or the like. The scaffold may be biodegradable or otherwise, for biodegradation in the body and in-growth by native cells, or for biodegradation in the environment after completion of bioremediation avoiding in each case the need for subsequent operation to remove the polymer.

In a further aspect of the invention there is provided an apparatus for use in the preparation of a polymer composite as hereinbefore defined. Suitably the apparatus comprises one or more pressure vessels adapted for temperature and pressure elevation and comprising means for mixing the contents. The pressure vessel may include means for depressurisation or for discharging of contents into a second pressure vessel at lower pressure. The apparatus comprises means for introduction of functioning matter, dense phase fluid and polymer whilst the vessel is pressurised, as commonly known in the art. Preferably the apparatus comprises a first chamber having cooling means for functioning matter, and a main pressure vessel for contacting dense phase fluid, functioning matter and dense phase fluid and polymer and means to discharge matter from first to main chamber and optionally from main to a second pressure vessel. Preferably discharging means are high velocity and comprising a pressure ram or the like.

In a further aspect of the invention there is provided a polymer composite as hereinbefore defined or a scaffold thereof for use as a support or scaffold for drug delivery, for use in bioremediation, as a biocatalyst or biobarrier for human or animal or plant matter, for use as a structural component, for example comprising the polymer and optional additional synthetic or natural metal, plastic, carbon or glass fibre mesh, scrim, rod or like reinforcing for medical or surgical insertion, for insertion as a solid monolith into bone or tissue, as fillers or cements for wet insertion into bone or teeth or as solid aggregates or monoliths for orthopaedic implants such as pins, or dental implants such as crowns etc.

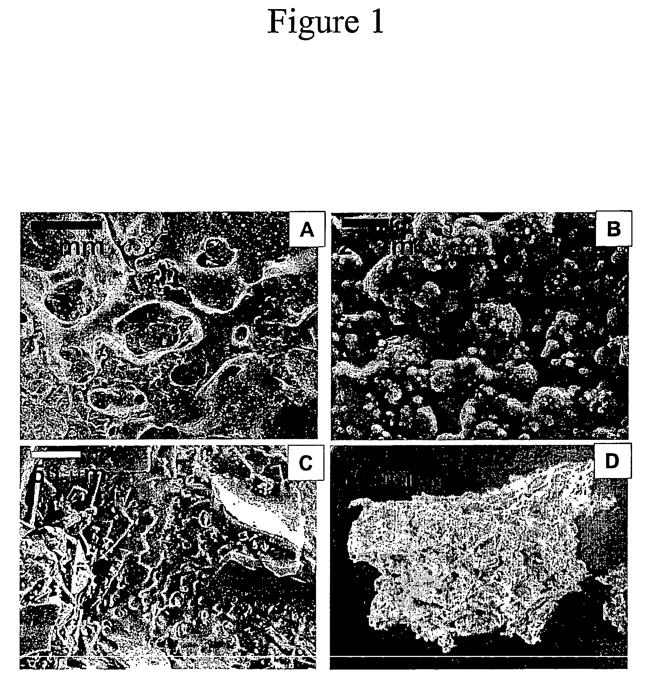

The invention is now illustrated in non limiting manner with reference to the following examples and Figures wherein
Figure 1 A - D shows scanning electron micrograph images of composites fabricated by the process of WO 98/51347 (Howdle et al) employed in the present invention; in Images A and B of an internal fracture surface of a monolith composite of calcium hydroxyapatite (40 wt%) and PLGA (60 wt%), at low magnification the distribution of calcium hydroxyapatite throughout the matrix and the production of pores is evident, at higher magnification the intimate mixing of guest particles and polymer is observed; in image C catalase (50% wt) is shown incorporated into a PLGA matrix (50%), micron scale pores in the polymer and the distinctive protein particle morphology are evident; in image D a high surface area microparticle composite (fluorescein (sodium salt) (8 wt%) and polycaprolactone (92 wt%)) are observed produced by direct atomisation, ie after fast depressurisation through an orifice.

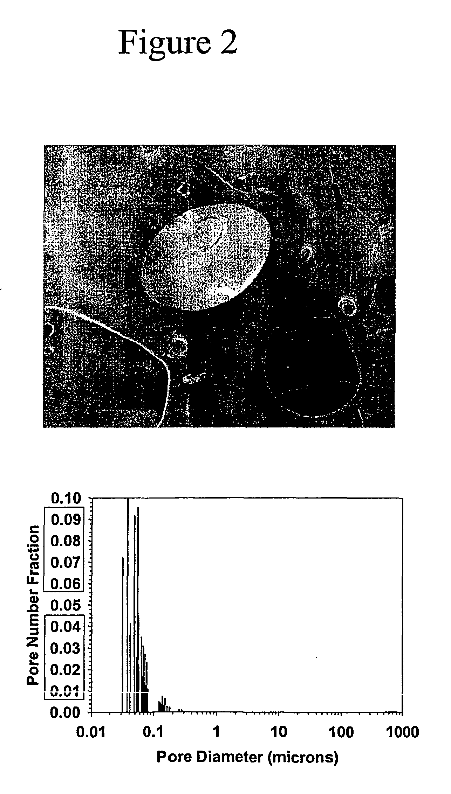

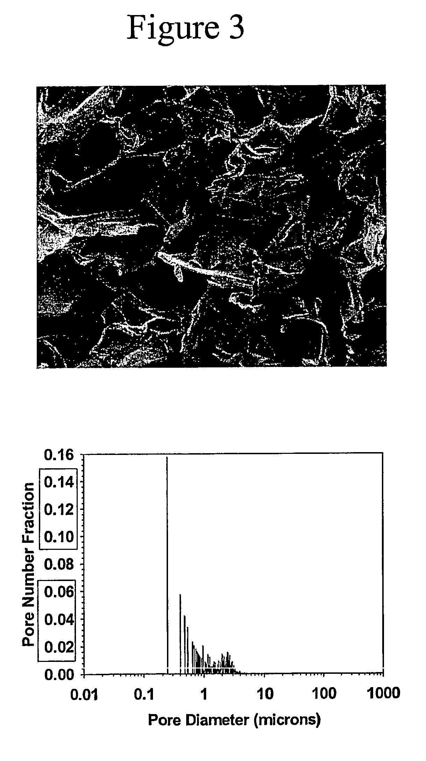

Figures 2 and 3 show scanning electron micrograph images and corresponding mercury porosimetry data for PLA composites fabricated by the process of WO 98/51347 (Howdle et al) employed in the present invention with control of PLA pore structure by changing de-pressurisation conditions; in Figure 2 the image shows presence of a small population of large pores obtained by depressurisation over a 2-hour period ("slow"); in Figure 3 the image shows an increase in porosity and a more heterogeneous distribution obtained by depressurisation over a 2-minute period ("fast"); data obtained by mercury porosimetry demonstrate that fine control over micropore distribution is achieved by changing only the de-pressurisation rate, with "slow" depressurisation creating pores in the 50 to 500 nm range, whilst "fast" depressurisation is strikingly different and creates pores in the 500 nm to 5 µm range

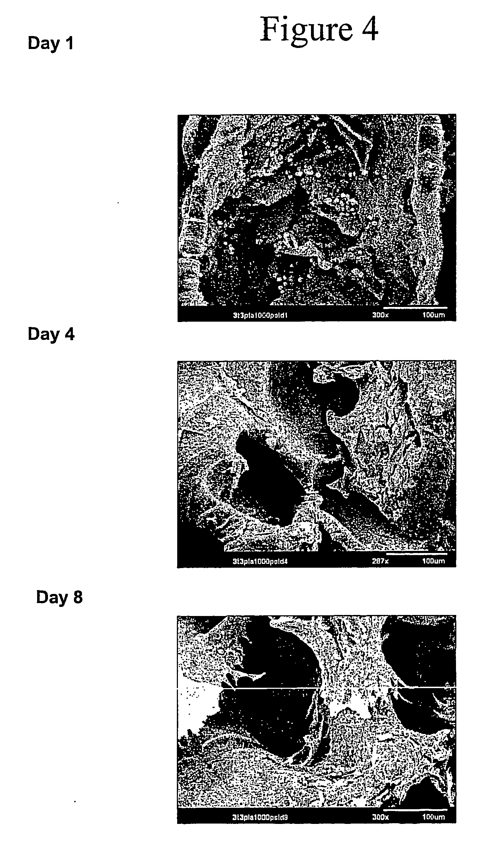

Figure 4 shows scanning electron micrograph images of cell-laden polymer composite fabricated according to the invention; viability of murine 3T3 fibroblasts is observed, with post processing incubation from Days 1 to 8, as described in the Examples showing spreading and attachment of cells and plaque formation

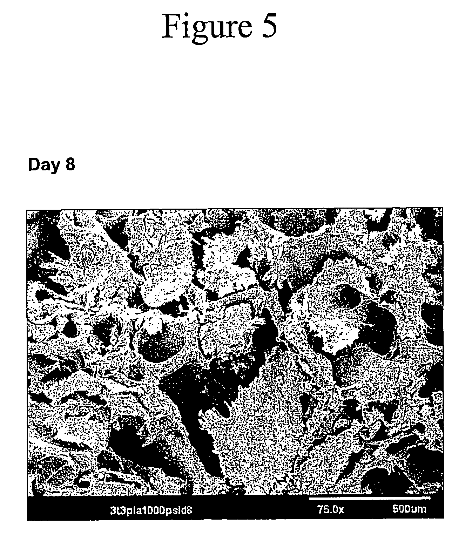

Figure 5 shows a scanning electron micrograph image of the cell-laden polymer composite of Figure 4 at Day 8 in greater magnification,

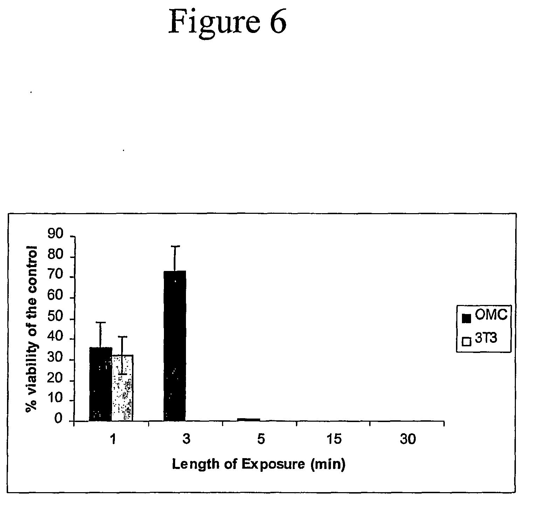

Figure 6 shows the viability of Ovine meniscal fibrochondrocytes (OMC) and 3T3 Fibroblasts after exposure to high pressure CO₂ for increasing time periods. Frozen cells were exposed to high pressure CO₂ at 68 x 10⁵ Nm⁻² (68 bar) and a temperature of 4°C for time periods of 1 to 30 minutes. Cells were then cultured and the viability of each cell type was measured.

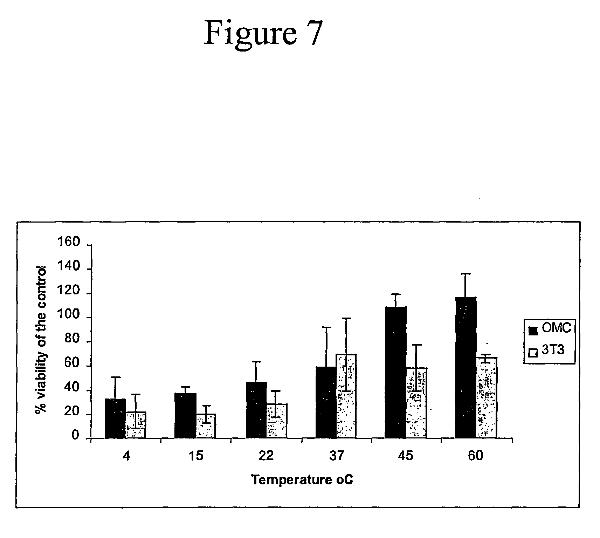

Figure 7 shows the effect of temperature on Ovine meniscal fibrochondrocytes (OMC) and 3T3 Fibroblasts. Frozen cells were exposed to high pressure CO₂ at 68 x 10⁵ Nm⁻² (68 bar), for an exposure time of 66 seconds at starting exposure temperatures from 4 to 60°C.

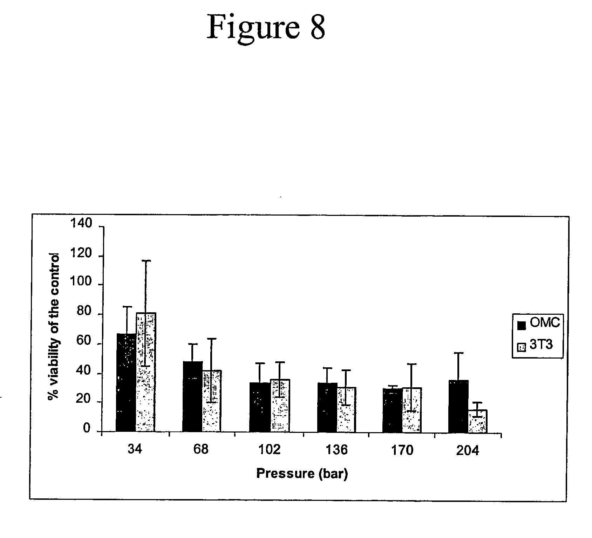

Figure 8 shows the effect of pressure on frozen Ovine meniscal fibrochondrocytes (OMC) and 3T3 Fibroblasts. Frozen cells were exposed to high pressure CO₂ between 34 and 204 x 10⁵ Nm⁻² (34 and 204 bar), the starting exposure temperature remained constant at 4°C and the exposure time was fixed at 66 seconds.

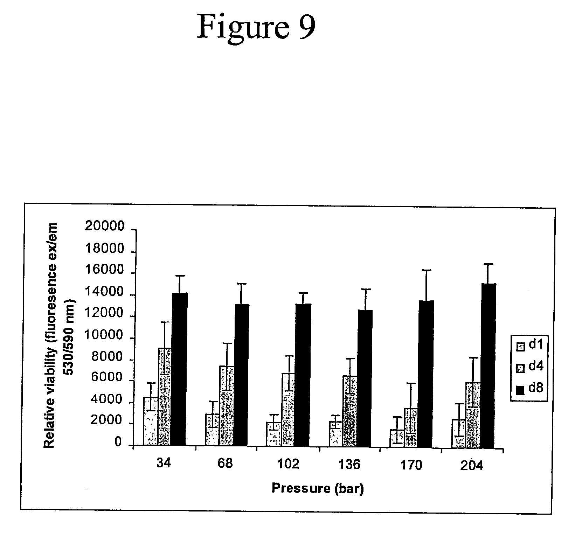

Figure 9 shows proliferation of primary chondrocytes over an 8 day period after exposure to a range of pressures of CO₂. Cells had been frozen and exposed to high pressure CO₂ between 34 and 204 x 10⁵ Nm⁻² (34 and 204 bar), at a starting exposure temperature of 4°C and exposure time of 66 seconds.

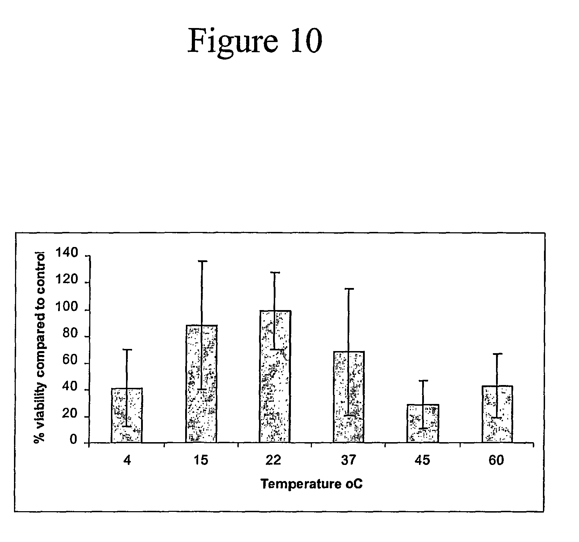

Figure 10 shows the effect of temperature on Ovine meniscal fibrochondrocytes (OMC). In this example cells were not frozen prior to exposure to high pressure CO₂ at 68 x 10⁵ Nm-² (68 bar), for an exposure time of 66 second, at starting exposure temperatures from 4 to 60°C.
Figure 1 shows the effect of pressure on Ovine meniscal fibrochondrocytes (OMC). These cells were not frozen prior to exposure to high pressure CO₂ between 34 and 204 x 10⁵ Nm⁻² (34 and 204 bar),; the starting exposure temperature remained constant at 4°C and the exposure time was fixed at 66 seconds.

### Materials and Methods

### Isolation of Cells

Ovine meniscal fibrochondrocytes (OMC) were isolated from the stifle joint of 3-6 month old lambs (slaughtered for the food chain) and the fibrochondrocytes isolated by digestion with pronase E (0.01 g/g cartilage; Merck, UK) for 3 hours at 37°C at 5%CO₂, followed by further digestion with collagenase (type II 0.02 g/g of cartilage; Lorne Laboratories, UK) for 18 hours at 37°C 5% CO₂. Cells were passed through a 70 µm filter (to remove undigested tissue), washed by centrifugation and expanded in culture in a humified atmosphere at 37°C, 5% CO₂ in DMEM supplemented with 10% FCS, 10% (v/v) foetal calf serum (FCS), 2 mM L-glutamine, 100 units/ml penicillin, 0.1 mg/ml streptomycin, and 0.25 µg/ml amphotericin B, 1x (v/v) non-essential amino acids (NEAA, 100x stock solution) 0,4 mM L-holine, and 75 µg/ml ascorbic acid-2-phosphate (OMC complete media).
Cells, were stored frozen until required when they were revived and cultured in OMC complete media as above. OMCs were cultured for no more than 3 days (to avoid dedifferentiation) and were used between passage number 3-6.

Murine 3T3 fibroblasts were obtained from the American Tissue and Culture Collection (ATCC; catalog. no. CCL-92) and maintained in monolayer culture, in a humidified atmosphere at 37°C, 5% CO₂ in DMEM supplemented with 10% (v/v) foetal calf serum, 2 mM L-glutamine, 100 units/ml penicillin, 0.1 mg/ml streptomycin, and 0.25 µg/ml amphotericin B (3T3 complete medium). 3T3s were used between passages 128-138.

Passaging and preparation of single cell suspensions for exposure to high pressure CO₂ conditions was achieved by enzymatic digestion using a 0.25 % (v/v) trypsin and 0.02% (w/v) EDTA solution in PBS. Cells were resuspended in 10% dimethylsulfoxide (DMSO), 0.1 M Hepes buffer pH 7.5, in FCS (modified freezing medium) and cell counts assessed using Trypan blue exclusion and a haemocytometer. Live cell numbers were used to determine final cell concentrations (1 x 10⁶ cells/ml stock; 200 µl or 2 x 10⁵ cells added to each well of the mould).

### Mould

The mould used for cell freezing and subsequent exposure to high pressure CO₂, was made by cutting a 48 well tissue culture plastic plate into 3 well pieces.

### Pressurisation of CO₂

For all experiments, pressurised CO₂ was pumped through the system by a Pickel pump (PM-1013; New Wave Analysis, Switzerland), through a back pressure regulator (model number BP-1580-81; Jasco, UK), to a 10 ml Thar cell autoclave (Thar Designs Inc, USA). For length of exposure experiments, the Thar cell was filled with high pressure CO₂ at a constant rate of 2.83 x 10⁵ Nm⁻² (2.83 bar/s) until 68x10⁵ Nm⁻² (68 bar) was reached. The vessel was held at pressure for the desired length of time (1, 3, 5,15, and 30 min) and the vessel vented until the pressure had returned to atmospheric (total time for filling and venting 37.5 s). For effect of pressure and temperature experiments, the autoclave was filled to desired pressure in 24 s (rate 2.83 x 105 Nm⁻² (2.83bar)/s), this pressure was held for 12 s, and the autoclave vented for 30 s (total time 66 s). Temperature was controlled using ice (4°C), cold water (15°C), room temperature (22°C), and a heating block (specially designed to hold the autoclave) for 37°C, 45°C, and 60°C.

### Conditions for frozen cells

For all experiments using frozen cells, cells were kept frozen in liquid N₂ vapour prior to exposure to high pressure CO₂. Following exposure, cells were stored on ice prior to being transferred to a 24 well tissue culture plastic plate with 1 ml warm complete media. Cells were incubated at 37°C, 5% CO₂ for 3 hours to allow for cell adhesion. The media was carefully removed so as to not disturb the cells and replaced with fresh (1 ml). Cells were incubated for a further 21 hours (day 1) prior to Alamar Blue assay. For cells cultured for more than one day, the Alamar Blue was removed at the end of the assay and replaced with 1 ml fresh complete media and cultured as described above.

### Viability testing of cells

Viability of cells following exposure to high pressure CO₂ and subsequent 24 hour culture in complete medium at 37°C, 5% CO₂, was determined using the Alamar Blue^{T.M.} assay using the following method. Cells were washed 3 times in PBS and incubated with 10% Alamar Blue^{T.M.} in Hank's balanced salt solution (pH 7) without phenol red (HBSS) for 90 minutes. Aliquots (100 µl) of Alamar Blue^{™} /HBSS were placed in a 96 well plate and the fluorescence measured at excitation wavelength 530 nm and emission wavelength 590 nm using a fluorescence plate reader (F2 Microplate Fluorescence, Absorbance and Luminescence System, Labtech, UK). Cell viability is either expressed as a measure of the fluorescence reading or as a percentage of control cells (treated in the same manner to test cells but with no exposure to high pressure CO₂) values.

### Preparation of scaffolds

Preparation of cultured scaffolds for SEM was carried out according to the following method,. Cultured scaffolds were washed in PBS and fixed in 1 ml 3% (v/v) glutaraldehyde at 4°C until required. Scaffolds were washed in PBS and fixed further in 1 ml 1% (v/v) osmium tetroxide in PBS for 2 hours. Scaffolds were washed with distilled water, dehydrated through a series of ethanol concentrations (25%, 50%, 75%, 90%, 95%, 100% [v/v] in distilled water), and chemically dried using hexamethyldisiazane (HMDS; Sigma, Poole UK). Dried scaffolds were sputter-coated with gold prior to analysis using a Philips 505 scanning electron microscope and Semicaps 200A v8.2 image analysis software.

### Example 1- Isolation of Functioning matter

Biological cells were prepared as either frozen or desiccated particles. A suspension of mouse 3T3 fibroblasts in 10% DMSO in FCS (foetal calf serum) containing 0.1M HEPES buffer pH 7 was frozen in a mould at -70°C for at least 2 hours (and up to one week) prior to exposure to dense phase CO₂. The resulting cell pellet was immersed in liquid nitrogen and then physically disrupted with a pestle and mortar to form particles with average diameters of between 50 and 1000 micron. DMSO acts as a cryopreservation agent allowing freezing and thawing without damage, as known in the art.

### Example 2a - Composite formation of Polymer with homogeneously dispersed isolated Functioning matter, by dense phase fluid processing within the autoclave.

Starting materials were poly (DL-luctic acid) (100 - 200 mg) and frozen mammalian cells obtained from Example 1. The frozen cells were seeded between two part-formed PLA slices and these were placed within an autoclave. The temperature was maintained at 4°C. Carbon dioxide was pumped into the autoclave until a pressure of 68x10⁵Nm⁻² (1000 psi, 68.03 bar) was achieved. A stirrer within the autoclave was rotated to combine the polymer and cell particles. In this case the stirrer was a helical impeller. After 10 seconds of exposure the pressure was rapidly released by opening a valve. The composite comprising polymer loaded with frozen cells was then removed from the autoclave and stored frozen in liquid nitrogen.

### Example 2b

In an alternative procedure for co-processing cells and polymer PLA was partially plasticised. The PLA was cut into discs (approximately 3 mm height), placed back into the glass tube and soaked with 3T3 cell suspension. This cell soaked scaffold was frozen overnight at -80°C prior to exposure to high pressure CO₂ conditions according to the method of Example.

### Examples 3 and 4

The method of Example 2 was repeated in each case with the autoclave maintained at 20°C and 37°C respectively for shorter contact times of 5 and 1 seconds.

### Example 5 - Viability testing

The composites from Examples 2 to 4 were allowed to thaw at room temperature, placed in cell culture media and incubated at 37°C with a 96% air/5% carbon dioxide atmosphere for 8 days. The composites are shown as scanning electron micrograph images at a fracture surface in Figure 3 A - F. Viability of the cells was assessed by confocal microscopy and SEM at Days 1, 4 and 8.

Cell survival was observed using an Alamar Blue assay, fluoresence correlating directly with cell viability. Cells were in a state for proliferation, as seen by the presence of confluent plaques after 8D.

In the case of composite from Example 2b, Scaffolds were cut so that they could be cultured in a 24 well plate and assayed for cell viability using the Alamar blue assay, using a scaffold processed in the same way but without cells as the assay blank. Following the Alamar blue assay, scaffolds were washed with PBS and a small piece cut and fixed in 3% glutaraldehyde at 4°C for at least overnight prior to further processing for SEM. The remaining scaffold was cultured further in complete medium and the above repeated at day 4 and 8.

### Example 6 - Viability of frozen functioning matter following exposure to high pressure CO₂

Ovine meniscal fibrochondrocytes (OMC) or 3T3 fibroblasts suspended in a modified freezing medium were frozen at -80°C overnight in a mould. These solid frozen cell pellets, each containing 2 x 10⁵ cells/ml, were exposed to high pressure liquid CO₂ (4°C and 68 x 10⁵ Nm⁻² (68 bar)) for increasing time periods and cultured for 24 hours in complete culture medium. The viability of each cell type measured using the Alamar Blue assay and quoted as a percentage of control cells is shown in Figure 6. Populations of both cell types retained over 30% viability after 1min of exposure. After 3 min exposure only the OMC population retained viability. After 5 min exposure both populations had died.

### Example 7 - Effect of Temperature on frozen functioning matter

OMC or 3T3 fibroblasts were exposed to high pressure liquid CO₂ according to the method of Example 6. The pressure was kept constant at 68 x 10⁵ Nm⁻² (68 bar), the total exposure time was fixed at 66 seconds and the starting exposure temperature was varied The cells were then cultured as described in Example 6 and the viability of each cell type measured using the Alamar Blue assay and quoted as a percentage of control cells. The results in Figure 7 show a trends of increased population viability, for both cell types, at higher temperatures. At 45 and 60°C the population viability approaches 100% of the control. Heating occurs as CO₂ is pumped rapidly into the vessel and results in a partial or complete thawing of the cell pellet at all starting temperatures. Then, as the pressure falls rapidly at the end of the exposure period a sharp fall in temperature may occur. It appears that at starting temperatures of 4, 15 and 22°C the Joules-Thompson cooling effect is sufficient to re-freeze the cell pellet with the expected loss of cell viability resulting from rapid ice crystal formation. In contrast at 37, 45 and 60°C the autoclave is partially insulated against the final temperature fall and the cells remain within the recently thawed suspension.

### Example 8 Pressure range for frozen functioning matter

OMC or 3T3 fibroblasts were exposed to high pressure liquid CO₂ according to the method of Example 6. The total exposure time and starting exposure temperature were fixed. The results shown in Figure 8 show cell viability after exposure to pressures between 34 x 10⁵ Nm⁻² and 204 x 10⁵ Nm⁻² (34 and 204 bar). For all experiments we observed variability in the cell survival data resulting from the effect of cryopreservation on overall viability of both cell type populations. However, accepting the inherent noise within the data from cryopreserved cells, there is a drop of population viability from 70% at 34x10⁵ Nm⁻² (34 bar) to less than 50% at greater pressures. The effect of Joules-Thompson heating and cooling complicates data interpretation as temperature, pressure and exposure time cannot be independently varied. However, the observation of cell survival up to 204 x 10⁵ Nm⁻² (204 bar) remains valid. A further step in the analysis of the primary chondrocytes post CO₂ exposure is shown in Figure 9 where cell proliferation over an 8 day period is evident. Similar rises in cell number were recorded after exposure to all pressures.

### Example 9 - Viability of non-frozen functioning matter following exposure to high pressure CO₂

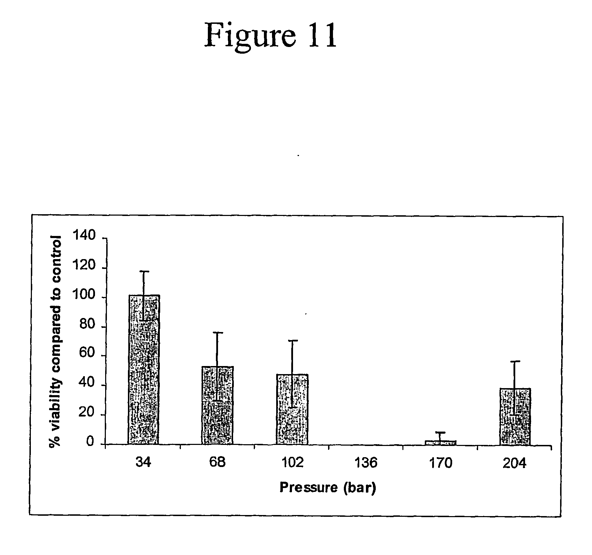

The cell suspension, in modified freezing medium, was diluted to the desired cell number and transferred to the moulds as described previously. Cells were kept on ice prior to and following exposure to high pressure CO₂ conditions and then transferred to complete cell culture media for culture. Cell viability was assessed using the Alamar Blue viability assay as described in Example 5. Figure 10 shows the effect of temperature on the viability of non-frozen OMC. At temperature of 12, 22 and 37°C over 60% viability was maintained but at higher and lower temperatures viability decreased to loss than 40%. Figure 11 shows the effect of pressure on the viability of non-frozen OMC. At 4°C, OMC population survival was unaffected by short exposure to 34 x 10⁵ Nm-² (34 bar) pressure of CO₂ but at pressures of 68 bar and above viability was compromised.

Further aspects and advantages of the invention will be apparent from the foregoing.

## Claims

1. A process for the preparation of a polymer composite loaded with functioning matter directly in one step, wherein functioning matter is selected from mammalian, plant and bacterial subcellular, cellular or multicellular matter, liposomes, and aggregates and mixtures thereof wherein the process comprises contacting a polymer substrate and functioning matter with a plasticising fluid or mixture of plasticising fluids comprising a liquid or gaseous fluid which is capable of plasticising polymer in its natural state or in supercritical, near critical, dense-phase or subcritical state having fluid density in the range 0.001 g/ml up to 10 g/ml under plasticising conditions to plasticise and/or swell the polymer and incorporate the functioning matter, and releasing the plasticising fluid to obtain the polymer composite, wherein contacting is for a short contact time of 20 milliseconds up to 5 minutes at a pressure in the range 1 x 10⁵ to 1 x 10⁸ Nm⁻² (1 to 1000 bar) and a temperature in the range -200 to +500°C, selected in manner that at least a proportion of functioning matter does not freeze or refreeze during processing, or if at a temperature at which freezing or refreezing may occur, that either matter is desiccated or a pressure constraint is applied whereby pressure is in a range having a maximum pressure less than 1 x 10⁸ Nm⁻² (1000 bar) throughout contact of functioning matter and plasticising fluid, whereby at least a proportion of functioning matter retains its function in the polymer composite.

2. A process as claimed in Claim 1 wherein contacting is with cryopreserved or non cryopreserved functioning matter and is conducted at a temperature at least in the range +4 to +35°C and maximum pressure less than 4 x 10⁷ Nm⁻² (400 bar) throughout contact of functioning matter and plasticising fluid.

3. A process as claimed in any of Claims 1 to 2 contacting is with cryopreserved or non cryopreserved functioning matter and is conducted at a temperature at least in the range +4 to +35°C and maximum pressure less than 2.75 x 10⁷ Nm⁻² (275 bar), for example in the range 5 x 10⁵ Nm⁻² to 75 x 10⁵ Nm⁻² (5 to 75 bar).

4. A process as claimed in any of Claims 1 to 3 wherein contacting is with carbon dioxide as plasticising fluid wherein freezing or refreezing may take place during processing at least in the temperature range +4 to +35°C, and a pressure constraint is applied.

5. A process as claimed in any of Claims 1 to 4 wherein at least 20% of functioning matter maintains function.

6. A process as claimed in any of Claims 1 to 5 wherein plasticising conditions comprise a pressure in the range 2 x 10⁵ Nm⁻² to 4x10⁷ Nm⁻² (2 to 400 bar), more preferably 5 x 10⁵ Nm ² to 2.65 x 10⁷ Nm⁻² (5 to 265 bar).

7. A process as claimed in any of Claims 1 to 6 wherein plasticising fluid includes carbon dioxide, di-nitrogen oxide, carbon disulphide, aliphatic C₂₋₁₀ hydrocarbons such as ethane, propane, butane, pentane, hexane, ethylene, and halogenated derivatives thereof such as for example carbon tetrafluoride or chloride and carbon monochloride trifluoride, and fluoroform or chloroform, -C₆₋₁₀ aromatics such as benzene, toluene and xylene, C₁₋₃ alcohols such as methanol and ethanol, sulphur halides such as sulphur hexafluoride, ammonia, xenon, krypton, or a mixture thereof.

8. A process as claimed in any of Claims 1 to 7 wherein functioning matter is present in an amount with respect to polymer of 1x10⁻¹²wt% to 99.9 wt%.

9. A process as claimed in any of Claims 1 to 8 wherein functioning matter is selected from mammalian, plant and bacterial cells including (subcellular) organelles and aggregates thereof including pancreatic islet or liver spheroids and the like; and liposomes as carrier of sensitive matter.

10. A process as claimed in any of Claims 1 to 9 wherein functioning matter is selected from mammalian and plant prokaryotic and eukaryotic cells and mixtures and aggregates thereof, and liposomes as carrier of protein or enzymes.

11. A process as claimed in any of Claims 1 to 10 wherein functioning matter comprises mammalian cells selected from fibroblasts, fibrochondrocytes, chondrocytes, bone forming cells such as osteoblasts and osteoclasts, bone marrow cells, hepatocytes, cardiomycytes, blood vessel forming cells, neurons, myoblasts, macrophages, microvascular endothelium cells and mixtures thereof and collagen, and liposomes.

12. A process as claimed in any of Claims 1 to 11 for the preparation of a polymer composite additionally comprising biofunctional material and loaded with functioning matter wherein the process comprises in a first stage contacting biofunctional material selected from drugs and veterinary products. agrochemicals, human and animal health products, human and animal growth promoting, structural or cosmetic products, and absorbent materials for poisons and toxins and polymer and a plasticising fluid or a mixture of plasticising fluids under plasticising conditions to plasticise and/or swell the polymer and incorporate the biofunctional material,

13. Process as claimed in any of Claims 1 to 12 wherein polymer is selected from: polyesters including poly(lactic acid), poly(glycolic acid), copolymers of lactic and glycolic acid, copolymers of lactic and glycolic acid with poly(ethylene glycol), poly(e-caprolactone), poly(3-hydroxybutyrate), poly(p-dioxanone), poly(propylono fumarate); poly (ortho esters); polyanhydrides; Poly(amino acids); polyacetals; polyketals; polyorthoesters; Polyphosphazenes; azo polymers; synthetic Non-biodegradable Polymers selected from: Vinyl polymers including polyethylene, poly(ethylene-co-vinyl acetate), polypropylene, poly(vinyl chloride), poly(vinyl acetate), poly(vinyl alcohol) and copolymers of vinyl alcohol and vinyl acetate, poly(acrylic acid) poly(methacrylic acid), polyacrylamides, polymethacrylamides, polyacrylates, Poly(ethylene glycol), Poly(dimethyl siloxone), Polyurethanes, Polycarbonates, Polystyrene and derivatives; and Natural Polymers selected from carbohydrates, polypeptides and proteins.

14. A polymer composite comprising a polymer loaded with functioning matter selected from mammalian, plant and bacterial subcellular, cellular or multicellular matter and aggregates and mixtures thereof obtainable by the process as defined in any of Claims 1 to 13, wherein at least 20% of functioning matter has retained function in the polymer composite.

15. A polymer composite comprising a polymer loaded with functioning matter selected from mammalian, plant and bacterial subcellular, cellular or multicellular matter and aggregates and mixtures thereof as defined in any of Claims 1 to 13 wherein functioning matter is non-estabiished, ie is directly loaded functioning matter, and is not proliferated, grown, adhered or otherwise modified post loading, at least 20% of which has retained function in the polymer composite.

16. A polymer composite as claimed in Claim 14 or 15 which is in granular or monolith form.

17. A polymer composite loaded with functioning matter selected from mammalian, plant and bacterial subcellular, cellular or multicellular matter and aggregates and mixtures thereof as claimed in any of Claims 14 to 16, additionally comprising biofunctional materials, selected from drugs and veterinary products, agrochemicals, human and animal health products, human and animal growth promoting, structural or cosmetic products, and absorbent materials for poisons and toxins, suitably sized and shaped for a desired application.

18. A polymer composite, a scaffold thereof or the process for the preparation thereof as claimed in any of Claims 1 to 17 for use as a support or scaffold for drug delivery, for use in bioremediation, as a biocatalyst or biobarrier for human or animal or plant matter, for use as a structural component, for example comprising the polymer and optional additional synthetic or natural metal, plastic, carbon or glass fibre mesh, scrim, rod or like reinforcing for medical or surgical insertion, for insertion as a solid monolith into bone or tissue, as fillers or cements for wet insertion into bone or teeth or as solid aggregates or monoliths for orthopaedic implants such as pins, or dental implants such as crowns.

## Patentansprüche

1. Verfahren zur Herstellung eines Polymerkomposits mit Funktionsmaterial direkt in einer Stufe, wobei das Funktionsmaterial ausgewählt ist aus von Säugern oder Pflanzen stammendem oder subzellulärem, zellulärem oder multizellulärem bakteriellen Material, aus Liposomen sowie Aggregaten und Mischungen aus den genannten Materialien, wobei das Verfahren eine Stufe umfasst, in der man ein Polymersubstrat und Funktionsmaterial mit einem plastifizierenden fluiden Stoff oder einer Mischung von plastifizierenden fluiden Stoffen einschließlich eines flüssigen oder gasförmigen fluiden Stoffes, der in seinem natürlichen Zustand oder in überkritischem, nahezu kritischem, durch eine dichte Phase **gekennzeichnet**em oder unterkritischem Zustand mit einer Dichte im Bereich von 0,001 g/ml bis zu 10 g/ml unter plastifizierenden Bedingungen das Polymer zu plastifizieren vermag, kontaktiert, um das Polymer zu plastifizieren und/oder quellen zu lassen und das Funktionsmaterial in das Polymer zu inkorporieren, und den plastifizierenden fluiden Stoff freisetzt, um das Polymerkomposit zu erhalten, wobei das Kontaktieren eine kurze Kontaktzeit von 20 Millisekunden bis zu 5 Minuten beansprucht und unter einem Druck im Bereich von 1 x 10⁵ bis 1 x 10⁸ Nm⁻² (1 bis 1.000 bar) sowie bei einer Temperatur im Bereich von -200 bis +500°C erfolgt, ausgewählt auf eine solche Weise, dass zumindest ein Teil des Funktionsmaterials während des Verfahrens nicht gefriert oder wieder gefriert oder, wenn eine Temperatur herrscht, bei der das Material gefrieren oder wieder gefrieren könnte, entweder das Material getrocknet oder ein Druck angelegt wird, wobei der Druck während des Kontakts von Funktionsmaterial und plastifizierendem fluiden Stoff in einem Bereich mit einem maximalen Druck von weniger als 1 x 10⁸ Nm⁻² (1.000 bar) liegt, wodurch zumindest ein Teil des Funktionsmaterials in dem Polymerkomposit seine Funktion beibehält.

2. Verfahren nach Anspruch 1, wobei die Kontaktierung mit kryopräserviertem oder nicht kryopräserviertem Funktionsmaterial durchgeführt wird, und bei einer Temperatur erfolgt, die mindestens im Bereich von +4°C bis +35°C liegt, und der maximale Druck während des Kontakts von Funktionsmaterial und plastifizierendem fluiden Stoff weniger als 4 x 10⁷ Nm⁻² (400 bar) beträgt.

3. Verfahren nach einem der Ansprüche 1 oder 2, wobei die Kontaktierung mit kryopräserviertem oder nicht kryopräserviertem Funktionsmaterial durchgeführt wird, und bei einer Temperatur erfolgt, die mindestens im Bereich von +4°C bis +35°C liegt, und der maximale Druck weniger als 2,75 x 10⁷ Nm⁻² (275 bar) beträgt, beispielsweise im Bereich von 5 x 10⁵ Nm⁻² bis 75 x 10⁵ Nm⁻² (5 bis 75 bar).

4. Verfahren nach einem der Ansprüche 1 bis 3, wobei die Kontaktierung mit Kohlendioxid als plastifizierendem fluiden Stoff erfolgt, in dem das Funktionsmaterial bei einer Verfahrenstemperatur von +4°C bis +35°C gefrieren oder wieder gefrieren kann, und ein Druck ausgeübt wird.

5. Verfahren nach einem der Ansprüche 1 bis 4, wobei mindestens 20% des Funktionsmaterials seine Funktion beibehält.

6. Verfahren nach einem der Ansprüche 1 bis 5, wobei die Plastifizierungsbedingungen einen Druck im Bereich von 2 x 10⁵ Nm⁻² bis 4 x 10⁷ Nm⁻² (2 bis 400 bar), vorteilhafter von 5 x 10⁵ bis 2,65 x 10⁷ Nm⁻² (5 bis 265 bar) einschließen.

7. Verfahren nach einem der Ansprüche 1 bis 6, wobei der plastifizierende fluide Stoff Kohlendioxid, Distickstoffoxid, Schwefelkohlenstoff, aliphatische C₂₋₁₀ Kohlenwasserstoffe wie z.B. Ethan, Propan, Butan, Pentan, Hexan, Ethylen und deren halognierte Derivate, wie z.B. Kohlenstofftetrafluorid oder -chlorid und Monochlortrifluormethan und Fluoroform oder Chloroform, C₆₋₁₀ Aromaten, wie z.B. Benzol, Toluol und Xylol, C₁₋₃ Alkohole, wie z.B. Methanol und Ethanol, Schwefelhalogenide, wie z.B. Schwefelhexafluorid, Ammoniak, Xenon, Krypton und Mischungen davon, einschließt.

8. Verfahren nach einem der Ansprüche 1 bis 7, wobei das Funktionsmaterial in einer Menge, bezogen auf das Polymer, von 1 x 10⁻¹² Gewichtsprozent bis 99,9 Gewichtsprozent zugegen ist.

9. Verfahren nach einem der Ansprüche 1 bis 8, wobei das Funktionsmaterial ausgewählt ist aus Säuger-, Pflanzen- und Bakterienzellen, einschließlich (subzellulären) Organellen, und Aggregaten davon, einschließlich Langerhansschen Inselzellen (pancreatic islets) oder Lebersphäroiden u.ä.; und Liposomen als Träger des empfindlichen Materials.

10. Verfahren nach einem der Ansprüche 1 bis 9, wobei das Funktionsmaterial ausgewählt ist aus prokaryotischen und eukaryotischen Zellen von Säugern und Pflanzen sowie Mischungen und Aggregaten davon, und Liposomen als Träger von Proteinen oder Enzymen.

11. Verfahren nach einem der Ansprüche 1 bis 10, wobei das Funktionsmaterial umfasst: Säugerzellen, ausgewählt aus Fibroblasten, Fibrochondrozyten, Chondrozyten, knochenbildenden Zellen, wie z.B. Osteoblasten und Osteoklasten, Knochenmarkzellen, Leberzellen, Herzzellen, blutgefäßebildenden Zellen, Neuronen, Myoblasten, Makrophagen, mikrovaskulären Endothelzellen und Mischungen davon, sowie Kollagen und Liposomen.

12. Verfahren nach einem der Ansprüche 1 bis 11 zur Herstellung eines Polymerkomposites mit Funktionsmaterial und zusätzlichem biofunktionellen Material, wobei das Verfahren eine erste Stufe umfasst, in der man ein biofunktionelles Material, ausgewählt aus Arzneimitteln und Veterinärprodukten Agrochemikalien Produkten für die menschliche und die tierische Gesundheit, das menschliche oder tierische Wachstum fördernden Produkten, strukturellen oder kosmetischen Produkten und Absorptionsmitteln für Gifte und Toxine, und ein Polymer und einen plastifizierenden fluiden Stoff oder eine Mischung von plastifizierenden fluiden Stoffen unter Plastifizierungsbedingungen miteinander kontaktiert, um das Polymer zu plastifizieren und/oder quellen zu lassen, und das biofunktionelle Material in das Polymer zu inkorporieren.

13. Verfahren nach einem der Ansprüche 1 bis 12, wobei das Polymer ausgewählt ist aus: Polyestern, einschließlich von Polymilch- oder -glykolsäure, Copolymeren von Milch- oder -Glykolsäure, Copolymeren von Milch- und -Glykolsäure mit Poly(ethylenglykol), Poly(e-caprolacton), Poly(3-hydroxybutyrat), Poly(p-dioxanon), Poly(propylenfumarat); Poly(orthoestern), Polyanhydriden; Poly(aminosäuren); Polyacetalen; Polyketalen; Polyorthoestern; Polyphosphazenen; Azopolymeren; synthetischen, biologisch nicht abbaubaren Polymeren, ausgewählt aus: Vinylpolymeren, einschließlich von Polyethylen, Poly(ethylen-co-vinylacetat), Polypropylen. Poly(vinylchlorid), Poly(vinylacetat), Polyvinylalkohol und Copolymeren von Polyvinylalkohol und Vinylacetat, Polyacrylsäure; Polymethacrylsäure, Polyacrylamiden, Polymethacrylamiden, Polyacrylaten, Poly(ethylenglykol), Poly(dimethylsiloxan), Polyurethanen, Polycarbonaten, Polystyrol und Derivaten; sowie natürlichen Polymeren, ausgewählt aus Kohlenhydraten, Polypeptiden und Proteinen.

14. Polymerkomposit, enthaltend ein Polymer mit Funktionsmaterial, ausgewählt aus von Säugern oder Pflanzen stammendem und subzellulärem, zellulärem oder multizellulärem bakteriellen Material sowie Aggregaten und Mischungen aus den genannten Materialien, erhältlich durch das in den Ansprüchen 1 bis 13 definierte Verfahren, wobei mindestens 20% des Funktionsmaterials seine Funktion in dem Polymerkomposit beibehalten hat.

15. Polymerkomposit, enthaltend ein Polymer mit Funktionsmaterial, ausgewählt aus von Säugern oder Pflanzen stammendem und subzellulärem zellulärem oder multizellulärem bakteriellen Material sowie Aggregaten und Mischungen aus den genannten Materialien, wie in einem der Ansprüche 1 bis 13 definiert, wobei das Funktionsmaterial nicht etabliert (non-established) ist, d.h. ein direkt inkorporiertes Funktionsmaterial ist, das nach dem Inkorporieren nicht vermehrt, gewachsen oder angehaftet (adhered) ist oder nach dem Inkorporieren anderweitig modifiziert wurde, und wobei mindestens 20% des Funktionsmaterials in dem Polymerkomposit seine Funktion beibehalten hat.

16. Polymerkomposit, wie in den Ansprüchen 14 oder 15 beansprucht, welches gekörnt oder als Monolith vorliegt.

17. Polymerkomposit, enthaltend ein Polymer mit Funktionsmaterial, ausgewählt aus von Säugern oder Pflanzen stammendem und subzellulärem, zellulärem oder multizellulärem bakteriellen Material sowie Aggregaten und Mischungen aus den genannten Materialien, wie in einem der Ansprüche 14 bis 16 beansprucht, und zusätzlich biofunktionelle Materialien enthaltend, welche ausgewählt sind aus Arzneimitteln und Veterinärprodukten, Agrochemikalien, Produkten für die menschliche und die tierische Gesundheit, das menschliche und tierische Wachstum fördernden, strukturellen oder kosmetischen Produkten, und Absorptionsmitteln für Gifte und Toxine, in geeigneter Weise dimensioniert und geformt für eine gewünschte Anwendung.

18. Polymerkomposit, ein Gerüst daraus oder das Verfahren zu deren Herstellung, wie in einem der Ansprüche 1 bis 17 beansprucht, zur Verwendung als Träger oder Gerüst zur Wirkstofffreisetzung, zur Verwendung als biologisches Heilmittel (bioremediation), als Biokatalysator oder Biobarriere für humanes, tierisches oder pflanzliches Material, zur Verwendung als strukturelle Komponente, die z.B. das Polymer und wahlweise weitere synthetische oder natürliche metallische, Kunststoff-, Kohlenstoff- oder Glasfasergitter oder -gewebe, Baumwollstoffe, Stäbe oder ähnliche verstärkende Elemente enthält, die für medizinische oder chirurgische Insertionen, zur Insertion als fester Monolith in Knochen oder Gewebe, als Füllstoff oder Zement für eine nasse Insertion in Knochen oder Zähne oder als feste Aggregate oder Monolithe für orthopädische Implantate, wie z.B. als Stifte, oder für dentale Implantate, wie z.B. Kronen, bestimmt sind.

## Revendications

1. Procédé pour la préparation d'un composite polymère chargé d'une substance fonctionnelle directement en une étape, selon lequel la substance fonctionnelle est sélectionnée parmi des substances mammifères, végétales, sous-cellulaires bactériennes, cellulaires ou multicellulaires, des liposomes, des agrégats et mélanges de ces derniers, procédé qui comprend la mise en contact d'un substrat polymère et d'une substance fonctionnelle avec un fluide plastifiant ou mélange de fluide plastifiant comprenant un liquide ou un fluide gazeux qui est capable de plastifier le polymère à son état naturel ou à l'état supercritique ou presque critique, en phase dense ou sous-critique avec une densité de fluide comprise entre 0,001 g/ml et 10 g/ml dans des conditions de plastification pour plastifier et/ou gonfler le polymère et incorporer la substance fonctionnelle et par libération du fluide plastifiant pour obtenir le composite polymère, procédé selon lequel la mise en contact est prévue pour une courte durée de contact de 20 millisecondes jusqu'à 5 minutes à une pression comprise entre 1x10⁵ et 1x10⁸ Nm⁻² (de 1 à 1 000 bars) et une température comprise entre -200°C et +500°C, sélectionnée de manière qu'au moins une proportion de la substance fonctionnelle ne gèle pas ou ne regèle pas pendant le traitement ou si à la température à laquelle le gel et le regel peut se produire soit cette substance est asséchée, soit une contrainte de pression est appliquée, la pression étant une valeur maximale inférieure à 1 x 10⁸ Nm⁻² (1 000 bars) pendant tout le contact de la substance fonctionnelle et du fluide de plastification, moyennant quoi au moins une proportion de la substance fonctionnelle conserve sa fonction dans le composite polymère.

2. Procédé selon la revendication 1, selon lequel la mise en contact s'effectue avec une substance fonctionnelle cryopréservée ou non cryopréservée et est conduite à une température comprise au moins entre +4 et +35°C et une pression maximale inférieure à 4 x 10⁷ Nm⁻² (400 bars) pendant le contact de la substance fonctionnelle et du fluide plastifiant.

3. Procédé selon l'une quelconque des revendications 1 à 2, selon lequel la mise en contact est effectuée avec une substance fonctionnelle cryopréservée ou non cryopréservée et est effectuée à une température comprise au moins entre +4 et +35°C et à une pression maximale inférieure à 275 x 10⁷ Nm⁻² (275 bars), par exemple comprise entre 5 x 10⁵ Nm⁻² et 75 x 10⁵ Nm⁻² (5 à 75 bars).

4. Procédé selon l'une quelconque des revendications 1 à 3, selon lequel la mise en contact est effectuée avec un dioxyde de carbone comme fluide plastifiant dans lequel la congélation ou la recongélation peut s'effectuer pendant le procédé au moins à une température comprise entre +4 et +35°C et une contrainte de pression est appliquée.

5. Procédé selon l'une quelconque des revendications 1 à 4, selon lequel au moins 20 % de la substance fonctionnelle conservent leurs fonctions.

6. Procédé selon l'une quelconque des revendications 1 à 5, selon lequel des conditions de plastification comprennent une pression comprise entre 2 x 10⁵ Nm⁻² et 4 x10⁷ Nm⁻² (2 à 400 bars), de préférence 5 x 10³ Nm⁻² et 2,65 x 10⁷ Nm⁻² (5 à 265 bars).

7. Procédé selon l'une quelconque des revendications 1 à 6, selon lequel le fluide plastifiant contient du dioxyde de carbone, de l'oxyde de diazote, du sulfite de carbone, des hydrocarbures aliphatiques C₂₋₁₀ tels que l'éthane, le propane, le butane, le pentane, l'hexane, l'éthylène et les dérivés halogénés tels que par exemple le tétrafluorure ou chlorure de carbone, le trifluorure monochlorure de carbone, le fluoroforme ou chloroforme, des hydrocarbures aromatiques C₆₋₁₀ tels que le benzène, le toluène et le xylène, les alcools C₁₋₃ tels que le méthanol et l'éthanol, les halogénures sulfurés tels que l'hexafluorure de sulfure, les ammoniacs, le xénon, le crypton et/ou des mélanges de ces derniers.

8. Procédé selon l'une quelconque des revendications 1 à 7, selon lequel la substance fonctionnelle est présente dans une quantité par rapport au polymère de 1x10⁻¹² % en poids à 99,9 % en poids.

9. Procédé selon l'une quelconque des revendications 1 à 8, selon lequel la substance fonctionnelle est sélectionnée parmi des cellules mammifères, végétales, bactériennes incluant des organelles (sous-cellulaires), des agrégats et des mélange de ces dernières incluant les îlots pancréatiques ou les sphéroïdes du foi et similaires ; et des liposomes en tant que porteurs de la substance sensible.

10. Procédé selon l'une quelconque des revendications 1 à 9, selon lequel la substance fonctionnelle est sélectionnée parmi des cellules mammifères et végétales procaryotiques ou eucaryotiques et des mélanges et agrégats de ces dernières et des liposomes en tant que porteurs de protéine ou d'enzymes.

11. Procédé selon l'une quelconque des revendications 1 à 10, selon lequel la substance fonctionnelle comprend des cellules mammifères sélectionnées parmi les fibroblastes, les fibrochondrocytes, les chondrocytes, les cellules osseuses telles que les ostéoblastes et les ostéoclastes, les cellules de moelle osseuse, les hépatocytes, les cardionycytes, les cellules formant les vaisseaux sanguins, les neurones, les myoblastes, les macrophages, les cellules endothéliales microvasculaires et des mélanges de ces dernières et du collagène et des liposomes.

12. Procédé selon l'une quelconque des revendications 1 à 11, pour la fabrication d'un composite polymère comprenant en plus une matière biofonctionnelle et chargé d'une substance fonctionnelle, selon lequel le procédé comprend dans une première étape la mise en contact du matériau bifonctionnel sélectionné parmi des médicaments et des produits vétérinaires, des produits de l'agrochimie et des produis de la santé animale, des produits cosmétiques ou structurels activateurs de croissance humaine et animale et des matériaux absorbants pour les poisons et les toxines et un polymère et un fluide plastifiant ou un mélange de fluides plastifiants dans des conditions plastifiantes pour plastifier et/ou gonfler le polymère et incorporer la matière biofonctionnelle.

13. Procédé selon l'une quelconque des revendications 1 à 12, selon lequel le polymère est sélectionné parmi des polyesters comprenant de l'acide poly(lactique), de l'acide poly(glycolique), des copolymères d'acide lactiques et glycoliques avec du pol(éthylène glycol), poly(e-caprolactone), poly(3-hydroxybutyrate); poly(p-dioxanone), poly(propylène fumarate); poly(ortho esters), polyanhadrides ; acides poly(aminés); polyacétales; polyorthoesters ; polyphosphazènes ; azo polymères ; polymères synthétiques non biodégradables sélectionnés parmi : les polymères vinyles incluant le polyéthylène, poly(éthyléne-co-vynyle-acétate), polypropylène, chlorure de poly(vinyle), acétate de poly(vinyle), (alcool) de poly(vinyle) et des copolymères d'alcool de vinyle et acétate de vinyle, acide de poly(acrylique), (acide poly(méthacrylique), polyacryliques ; polyméthacrylamides, polyacrylates, poly(éthylène glycol), poly(diméthyle siloxane), polyuréthanes, polycarbonates, polystyrène et les dérivés ; et des polymères naturels sélectionnés parmi les carbonhydrates, les polypeptides et les protéines.

14. Composite polymère comprenant un polymère chargé de substance fonctionnelle sélectionnée parmi des substances multicellulaires ou cellulaires mammifères, végétales et bactériennes sous-cellulaires, des agrégats et mélanges de ces dernières pouvant être obtenus par le processus tel que défini selon l'une quelconque des revendications 1 à 13, selon lequel au moins 20 % de la substance fonctionnelle ont conservé la fonction dans le composite polymère

15. Composite polymère comprenant un polymère chargé d'une substance fonctionnelle sélectionnée parmi des substances multicellulaires ou cellulaires mammifères, végétales et bactériennes sous-cellulaires, des agrégats et mélanges de ces dernières tel que défini dans l'une quelconque des revendications 1 à 13, dans lequel la substance fonctionnelle n'est pas établie, c'est-à-dire est une substance fonctionnelle directement chargée, et n'est pas proliférée, ne subit pas de croissance et d'adhésion ou n'est pas modifiée d'une autre manière après le chargement, dont au moins 20 % ont une fonction retenue dans le composite polymère.

16. Composite polymère selon la revendication 14 ou 15, qui est de forme granulée ou monolithe.

17. Composite polymère chargé d'une substance fonctionnelle sélectionnée parmi des substances multicellulaires ou cellulaires mammifères, végétales et bactériennes sous-cellulaires, des agrégats et des mélanges de ces dernières, selon l'une quelconque des revendications 14 à 16, comprenant en plus des matériaux biofonctionnels, sélectionnés parmi des médicaments et des produits vétérinaires, des produits agrochimiques, et de la santé humaine et animale et des produits structurels et cosmétiques de la promotion de croissance humaine et animale et des matériaux absorbants pour les poisons et les toxines, de dimensions et de formes appropriées pour une application recherchée.

18. Composite polymère, une armature ou le procédé de préparation de ce dernier, selon l'une quelconque des revendications 1 à 17, pour un usage en tant que support ou vecteur pour la délivrance de médicaments, pour un usage en bioremédiation en tant que biocatalyseur ou barrière biologique pour des substances animales ou végétales, en tant que composant structurel, comprenant par exemple la maille, la gaze, la tige ou équivalent en polymère et métal, plastique, fibre de carbone ou de verre supplémentaires en option, renforçant l'insertion médicale ou chirurgicale, pour une insertion en tant que monolithe solide dans un os ou un tissu, en tant que vecteurs ou ciments pour une insertion par voie humide dans l'os ou la dent ou en tant qu'agrégats solides ou monolithes pour des implants orthopédiques tels que des broches ou des implants dentaires tels que des couronnes.
